(19)

Europäisches
Patentamt

European
Patent Office

Office européen
des brevets

(11)    **EP 2 688 912 B1**

(12)                      **EUROPEAN PATENT SPECIFICATION**

(45) Date of publication and mention
of the grant of the patent:
**09.11.2016   Bulletin 2016/45**

(21) Application number: **12760094.8**

(22) Date of filing: **22.03.2012**

(51) Int Cl.:
***C07K 19/00*** *(2006.01)*      ***G01N 33/542*** *(2006.01)*

(86) International application number:
**PCT/SG2012/000098**

(87) International publication number:
**WO 2012/128722 (27.09.2012 Gazette 2012/39)**

(54) **RECOMBINANT PROTEIN BIOSENSORS AND A METHOD FOR DETECTING THE PRESENCE OF AN ANALYTE MOLECULE**

REKOMBINANTE PROTEINBIOSENSOREN UND VERFAHREN ZUR ERKENNUNG DER PRÄSENZ EINES ANALYTMOLEKÜLS

BIODÉTECTEURS DE PROTÉINES RECOMBINANTES ET PROCÉDÉ POUR DÉTECTER LA PRÉSENCE D'UNE MOLÉCULE D'ANALYTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2011  SG 201102087**

(43) Date of publication of application:
**29.01.2014   Bulletin 2014/05**

(73) Proprietor: **Agency for Science, Technology and Research**
**Singapore 138632 (SG)**

(72) Inventors:
• **NIRANTAR, Saurabh Rajendra**
**Singapore 138648 (SG)**
• **GHADESSY, Farid John**
**Singapore 138648 (SG)**

(74) Representative: **Viering, Jentschura & Partner mbB**
**Patent- und Rechtsanwälte**
**Kennedydamm 55 / Roßstrasse**
**40476 Düsseldorf (DE)**

(56) References cited:
**WO-A1-00/03727          WO-A2-02/090987**
**WO-A2-03/068945        WO-A2-2007/051207**
**WO-A2-2008/109161    WO-A2-2009/117726**
**WO-A2-2010/088411    US-A1- 2005 026 234**
**US-B2- 6 783 958**

• **SAURABH R. NIRANTAR ET AL: 'A generic scaffold for conversion of peptide ligands into homogenous biosensors' BIOSENSORS AND BIOELECTRONICS vol. 47, 01 September 2013, NL, pages 421 - 428, XP055271159 DOI: 10.1016/j.bios.2013.03.049 ISSN: 0956-5663**

**Description**

FIELD OF THE INVENTION

The present invention relates to recombinant protein biosensors and methods for detecting the presence of an analyte molecule, in particular, recombinant protein biosensors and methods for detecting the presence of an analyte molecule using analyte binding molecules.

BACKGROUND OF THE INVENTION

One-step homogenous biosensors have the potential to significantly simplify and expedite analyte detection procedures as tedious washing steps or secondary detection reagents (like HRP labeled antibodies) that are the norm with current procedures such as ELISA are not required. However developing such biosensors has been a considerable undertaking, usually requiring starting from scratch for each and every analyte of interest. On the other hand, a wide variety of protein domains known to bind analytes of interest with high specificity and sensitivity exist, ranging from monoclonal antibodies and derivative single-chain variable fragments (scFvs) to artificial binding molecules based on scaffolds such as fibronectin to peptide aptamers.

WO 00/03727 A1 discloses FRET biosensors of glucose wherein signal production is based on the conformational change the glucose/galactose binding protein ("GGBP") undergoes upon binding to glucose.

However, there remains a need to provide a modular and predictable mechanism to convert the above-mentioned binders into analyte responsive biosensors, which would greatly facilitate many laboratory procedures.

SUMMARY OF THE INVENTION

In a first aspect, the invention relates to a fusion protein comprising a structure

$$(A_n\text{-}B\text{-}A_x\text{-}C\text{-}A_m)_y$$

wherein

    each A independently comprises a peptide or protein domain that binds an analyte of interest,
    wherein A comprises an antibody, antibody fragment, peptide aptamer, peptide antigen, or peptide antigen fragment, wherein the peptide antigen or peptide antigen fragment preferably comprises an antigenic determinant or epitope;
    B is an enzyme or has enzymatic activity, and comprises an entity that can produce a detectable signal;
    C is an inhibitor of B, and comprises an entity that, when A is not bound to the analyte of interest, binds to B and modulates the signal production by B;
    n and m are each independently 0 or at least 1 ;
    x is an integer of 0 or at least 1 ;
    y is an integer of at least 1 ;
    "-" represents a covalent bond or a linker comprising or consisting of one or more amino acids;
    wherein B and C each independently comprises a peptide or protein domain,

wherein the length of the linker between the peptides or protein domains is selected such that it allows the interaction of B and C when A is not bound to the analyte of interest, and it prevents the interaction of B and C when A is bound to the analyte of interest.

In a second aspect, a method for detecting a presence or amount of an analyte molecule is provided. The method comprises contacting a fusion protein as defined in accordance to various embodiments of the present invention with the analyte molecule under conditions that allow binding of A to the analyte molecule; and detecting the presence of the analyte molecule by determining a signal produced by the fusion protein:analyte complex.

In a third aspect, a kit for detecting a presence of an analyte molecule or an amount of analyte is provided. The kit comprises a fusion protein in accordance to various embodiments of the present invention.

**BRIEF DESCRIPTION OF THE DRAWINGS**

In the following description, various embodiments of the invention are described with reference to the following drawings, in which:

Figure 1 shows (a) a schematic depiction of a biosensor comprising the fusion protein Tem1 lactamase-HIV p17 epitope (EKIRLR)-beta lactamase inhibitor protein (BLIP); and (b) the rate of substrate turnover, as measured by nitrocefin absorbance of the HIV p17 epitope biosensor in the presence of various analytes, in accordance to various embodiments;

Figure 2 shows OD492 increase in the presence of various analytes for the biosensor mentioned in Figure 1, in accordance to various embodiments;

Figure 3 shows the response of DO1 biosensor (as in Figure 1, but with the peptide epitope of DO1 antibody between Tem1 and BLIP instead of EKIRLR peptide) monitored by nitrocefin cleavage absorbance at OD 492 nm, in accordance to various embodiments;

Figure 4 shows substrate turnover for various analytes being added to DO1 biosensor, in accordance to various embodiments;

Figure 5 shows a schematic representation of the rigidification of Mdm2 upon binding its cognate analytes, in accordance to various embodiments;

Figure 6 shows the response to Mdm2 biosensor based on triplicates of various analytes, in accordance to various embodiments;

Figure 7 shows images of visual appearance of the reaction of Figure 6, in accordance to various embodiments;

Figure 8 shows the response to Mdm2 biosensor, in accordance to various embodiments;

Figure 9 shows images for JM109 cells being transformed with expression plasmids encoding indicated biosensor (Kan resistance cassette) and DO1 ScFv (Chlor resistance cassette), in accordance to various embodiments;

Figure 10 shows a schematic representation of the coiled-coil binding for fluorophore-quencher configuration, in accordance to various embodiments;

Figure 11 shows the response of p53 short peptide against specific DO1 and non-specific (bp53) antibodies, in accordance to various embodiments; and

Figure 12 shows images of samples of Figure 11 excited by fluorescence, in accordance to various embodiments.

## DETAILED DESCRIPTION OF THE INVENTION

The following detailed description refers to, by way of illustration, specific details and embodiments in which the invention may be practiced. These embodiments are described in sufficient detail to enable those skilled in the art to practice the invention. Other embodiments may be utilized and structural, and logical changes may be made without departing from the scope of the invention. The various embodiments are not necessarily mutually exclusive, as some embodiments can be combined with one or more other embodiments to form new embodiments.

In a first aspect, a fusion protein comprising a structure of

$$(A_n\text{-}B\text{-}A_x\text{-}C\text{-}A_m)_y$$

wherein each A independently comprises a peptide or protein domain that binds an analyte of interest, wherein A comprises an antibody, antibody fragment, peptide aptamer, peptide antigen, or peptide antigen fragment, wherein the peptide antigen or peptide antigen fragment preferably comprises an antigenic determinant or epitope; B is an enzyme or has enzymatic activity, and comprises an entity that can produce a detectable signal; C is an inhibitor of B, and comprises an entity that, when A is not bound to the analyte of interest, binds to B and modulates the signal production by B; n and m are each independently 0 or at least 1; x is an integer of 0 or at least 1; y is an integer of at least 1, wherein B and C each independently comprises a peptide or protein domain, wherein the length of the linker between the peptides or protein domains is selected such that it allows the interaction of B and C when A is not bound to the analyte of interest, and it prevents the interaction of B and C when A is bound to the analyte of interest.

The symbol "-"represents a covalent bond or a linker comprising or consisting of one or more amino acids.

In the context of various embodiments, the term "fusion" or "fusion protein" refers to a covalent linkage of two or more proteins or fragments thereof via the respective peptide backbones. The linkage may also be co-linear or co-translational. A fusion protein is an artificial protein or polypeptide derived from fusing the two or more proteins or fragments thereof. In some examples, the proteins and fragments thereof may be from different sources.

As used herein, the term "domain" with reference to a protein may refer to an independently folding peptide structure that may naturally be part of a larger protein. For example, a domain in the sense of the present invention may include one or more amino acid stretches that have a secondary, optionally a tertiary and optionally a quaternary structure and fold independently from other parts of the protein that may not be present in the isolated domain.

The term "structure" refers to a chemical structure.

The term "binds" refers to an interaction between a target and a potential binding component wherein the potential binding component preferentially associates with the target. The association may be measured by a level. The term "binding component" refers to a compound that has a statistically significant association with a target molecule. The binding may generally be specific or non-specific. In various embodiments, the binding of the protein or peptide domains may be specific to the analyte of interest.

The term "entity" may refer to but is not limited to a chemical moiety, such as a peptide, or a protein, or a protein domain, or organic molecule. The entity may comprise or consist of a chromophore or fluorophore a quenching group, wherein this quenching group can absorb emission (or excitation) energy from a fluorophore or chromophore, usually in spatial proximity, and thus quench the signal generated by the fluorophore or chromophore.

As used herein, the term "produce" may interchangeably be referred to generate, send, give off, give or emit. The term "detectable signal" refers to a signal that can be detected or measured directly or indirectly. For example, the detectable signal may be detectable or measurable by physical, spectroscopic, photochemical, biochemical, immunochemical or chemical means. The detectable signal may be produced directly or indirectly by reaction or interaction with a suitable conjugate, for example, a substrate. The detectable signal may be an "indicator molecule".

In the context of various embodiments, the term "modulates" refers to change. The change may include stimulation. For example, the signal may be modulated by an increase in the signal or a decrease in the signal.

The term "linker" refers to a linking component or a spacing moiety which can covalently or non-covalently link a compound to a solid support or a protein domain or another chemical entity. Linkers may be selected based on their length, chemical stability, affinity, flexibility.

The term "amino acid" refers to naturally occurring and artificially produced amino acids, and also amino acid analogs and amino acid mimetics that function in a similar manner to the naturally occurring amino acids. Amino acid analogs refer to compounds that have the same basic chemical structure as the naturally occurring amino acids.

In one example, the one or more amino acids may refer but are not limited to one amino acid, two amino acids, three amino acids, four amino acids, five amino acids, six amino acids, seven amino acids, eight amino acids, nine amino acids, ten amino acids, or twenty amino acids.

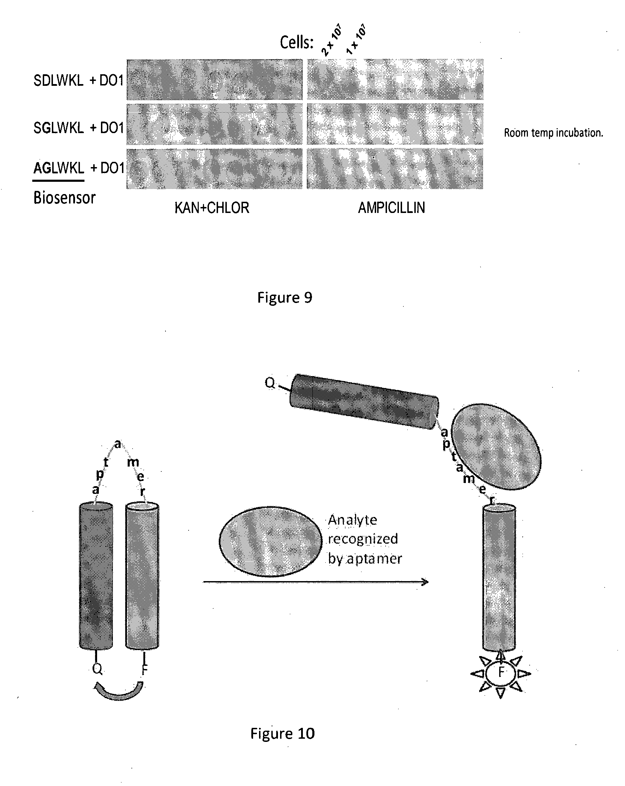

The use of chemical linkers allows to connect the fluorophore/quencher to the conditionally fluorescent peptides (eg. as shown in Figure 10).

B and C each independently comprise a peptide or protein domain. For example, B may be a peptide or protein domain coupled to a substance that can produce the detectable signal.

B is an enzyme or hasenzymatic activity. The enzyme may be a catalytic peptide. The enzyme may be capable of providing a convenient read-out. For example, the enzyme may be but is not limited to lactases, catalases, amylases, beta-lactamases, cephalosporinases, penicillinases, cephalosporinases, and carbenicilliniases. In various other embodiments, B may be beta-galactosidase or alkaline phosphatase.

C isan inhibitor of the enzyme, for example a protein inhibitor. The inhibitor may be a competitive inhibitor or an allosteric inhibitor. Binding of the inhibitor to the enzyme is reversible. For example, the protein inhibitor may be but is not limited to a protein inhibitor such as alpha 1 -antitrypsin, CI -inhibitor, antithrombin, alpha 1 -antichymotrypsin, plasminogen activator inhibitor- 1, neuroserpin, or a beta-lactamase inhibitor protein (BLIP), sulbactam, and tazobactam.

The length of the linker between the peptides or protein domains is selected such that it allows the interaction of B and C when A is not bound to the analyte of interest, and it prevents the interaction of B and C when A is bound to the analyte of interest. The analyte of interest interchangeably refers to an analyte under test.

In one embodiment, the binding of B and C may be impaired when all A moieties of the fusion protein are bound to an analyte, optionally the same analyte.

In specific embodiments, the fusion protein may comprise the structure of $(B-A-C)_y$. The structure is obtained when m and n are each independently 0; and x is 1.

In other embodiments, B may comprise a chromophore or fluorophore and C may comprise a substance that absorbs emission energy of the chromophore or fluorophore (quencher) or vice versa.

In one embodiment, B is a chromophore or fluorophore. In another embodiment, B may be conjugated to the chromophore or fluorophore with B being a peptide, protein or protein domain. B may also be a fluorescent protein or peptide.

In one embodiment, C is a substance that absorbs emission energy of the chromophore or fluorophore. In another embodiment, C may be conjugated to such a substance that absorbs emission energy of a chromophore or fluorophore. In still another embodiment, C may be fluorescent peptide or protein, for example a conditionally fluorescent peptide. As used herein, the term "conjugated" means to be joined together, to be coupled, or to act or operate as if joined. Usually, conjugation occurs by covalent linkage or ionic interaction.

In one embodiment, B or C may comprise or be a fluorophore and the other is or comprises a substance that absorbs emission energy of the fluorophore, i.e.,e a quencher. In an embodiment, B may comprise a fluorophore and the substance that absorbs emission energy of the chromophore (or fluorophore) may be a quencher.

For example, the fluorophore may be but is not limited to fluorescein, 5-((2-Aminoethyl)amino)naphthalene-1-sulfonic acid (EDANS), isothiocyanate, coumarin, cyanine and rhodamine. Examples of a quencher may be but not limited to dark quencher, dimethylaminoazosulfonic acid, black hole quenchers, dabcyl, and Qxl quencher. More specifically, dabcyl may be a quencher to inhibit fluorophores in the context of conditionally fluorescent peptides (for example, as illustrated in Figure 10).

In other embodiments, the fluorophore may be green fluorescent protein (GFP). If GFP or derivatives thereofare used as a read-out molecule, then another fluorescent protein such as but not limited to red fluorescent protein (RFP) may be used as the interaction partner. In such a case, the detection makes use of the so-called fluorescence resonance energy transfer (FRET) technique, where the one fluorescent moiety, when in close proximity to the other (in the absence of analyte), will absorb photons emitted by the other fluorophore via Fluorescence Resonance Energy Transfer (FRET) and re-emit a longer wavelength photon. In case of using GFP and RFP, for example, the GFP emission photons are not seen when RFP is in close proximity; as RFP is inhibiting GFP emission so that only RFP emission is detected. When analyte is added and RFP is moved away from GFP more of the GFP emission photons will be detected and a GFP signal is detectable. In this way, a FRET acceptor such as RFP may also be considered an inhibitor of a FRET donor (such as GFP). However, in preferred embodiments of the invention not both, B and C, comprise or are fluorophores and the detection principle is not based on FRET.

In various embodiments, when more than one A is present in the fusion protein, each A may be the same, or two or more A may be different. In one example, the two or more A may be arranged to bind to the same analyte.

In various embodiments, A may comprise an antibody, antibody fragment, peptide aptamer, peptide antigen, or peptide antigen fragment.

As used herein, the term "antibody" may be used in the broadest sense and covers polyclonal antibodies, monoclonal antibodies, multispecific antibodies, single domain antibodies, and phage antibodies. Antibodies may refer to fragments of antibodies. The term "antibody fragment" means a portion of the full length antibody, generally the antigen binding or variable region thereof. For example, an antibody fragment may include single chain antibodies (scFv) or binding fragment (Fab). Antibodies may be interchangeably referred to as immunoglobulin. Varieties of antibodies may be, for example, IgA, IgD, IgE, IgG and IgM. Examples of antibodies may be but not limited to anti-HIV p17 epitope, p53 (DO-1) monoclonal antibody, and anti c-myc antibody.

In some examples, A may comprise naturally occurring ligands or interacting partners. For example, Mdm2 may be used as A for the detection of p53.

The "peptide aptamer" may be a combinatorial protein reagent that binds to target proteins with a high specificity and a strong affinity. For example, the peptide aptamer may inhibit the function of a protein in vivo.

The term "antigen" generally refers to a molecule capable of being bound by an antibody. For example, an antigen may be but is not limited to pathogen derived proteins/molecules, molecules of medical interest such as insulin, hcG, etc. In one embodiment, the antigen or antigen fragment may comprise or consist of an antigenic determinant or epitope.

In some embodiments, x may be the integer 1 or 2. In other embodiments, x may be an integer of more than 2. For example, when x =1, the fusion protein may comprise the structure of $(A_n\text{-}B\text{-}A\text{-}C\text{-}A_m)_y$. When x =2, the fusion protein may comprise the structure of $(A_n\text{-}B\text{-}A\text{-}A\text{-}C\text{-}A_m)_y$. The structure of the fusion protein is given in N-terminal to C-terminal orientation.

The term "C-terminal" or "C-terminus" used herein is not equivalent to the symbol "C" in the structure of the fusion protein.

Further described herein is that the respective positions of B and C may be exchanged within the fusion protein such that the fusion protein comprises a structure of $(A_n\text{-}C\text{-}A_x\text{-}B\text{-}A_m)_y$.

In various embodiments, the binding of C to B may decrease or inhibit signal production by B.

In other embodiments, C may initiate or increase signal production by B upon binding. Such initiation or increase may be upon the binding of B and C. As such, C may be or may involve an activator or an enhancer. For example, an activator may be an enzyme activator, which is a molecule that binds to an enzyme and increases its activity. For illustrative purposes, an example of an enzyme activator may be the fragment of beta-galactosidase and variants thereof, which activates the $\Omega$ fragment. Other enzyme activators may work in a similar manner. For example, an enhancer may be an enzyme enhancer, which can bind to a non-active site and cause a conformation change which enhances enzyme function.

In one embodiment, B may be beta lactamase or homologs, fragments and variants thereof, wherein the homology, fragments and variants at least partially retain enzymatic activity. In other embodiments, B may be various beta lactamases or homologs, fragments and variants thereof, wherein the homology, fragments and variants at least partially retain enzymatic activity. In one specific embodiment, B is the beta lactamase TEM1 or variants thereof. "Variant", as used herein, refers to a protein that differs from a consensus sequence or the accepted wildtype sequence by at least one amino acid variation. The variant may be but is not limited to a natural variant, or a M69L variant, or a E104K variant. The beta lactamase TEM1 may comprise the amino acid sequence set forth in SEQ ID NO: 1 (UniProtKB accession number Q5QJI7). In one specific embodiment, the beta lactamase TEM1 may comprise a homolog or fragment or variant of the amino acid sequence set forth in SEQ ID NO: 1.

"Homologs", as used herein, refer to two proteins that have similar amino acid sequence. Homologs include orthologs, or paralogs. "Fragments", as used herein, refer to a portion of amino acid sequence, that is, a polypeptide comprising fewer than all of the amino acid residues of the protein.

Generally, as used herein, the term "beta lactamase" includes multiple beta lactamases, for example, any of Class A beta lactamases, Class B beta lactamases, Class C beta lactamases, and/or Class D beta lactamases. In one embodiment, the beta lactamase is a Class A beta lactamase, such as, for example, TEM1.

In another embodiment, C may be beta lactamase inhibitor protein (BLIP) or BLIP-I or BLIP-II or fragments, homologs and variants thereof that retain at least partially the binding activity for beta lactamase. In one specific embodiment, C is a beta lactamase inhibitor protein (BLIP) or variants thereof. The terms "variant", "homologs" and "fragments" are as defined above. The BLIP may have the amino acid sequence set forth in SEQ ID NO: 2 (UniProtKB accession number Q18BP3). In one specific embodiment, the BLIP may comprise a homolog or fragment or variant of the amino acid sequence set forth in SEQ ID NO: 2. In an embodiment, C may comprise molecules with similar function such as BLIP-1 and BLIP-II.

In various embodiments, A may be an antigenic peptide. The antigenic peptide may comprise or may consist of an amino acid sequence selected from the group consisting of those set forth in SEQ ID NOs: 3, 8-10, 12-13.

In one embodiment, the symbol "-" may represent a linker comprising more than one amino acids and wherein the linker peptide forms a coiled coil structure.

As used herein, the term "coiled coil" may refer to a peptide or protein sequence usually with a contiguous pattern of hydrophobic residues spaced 3 or 4 residues apart and assembles (or folds) to form a multi-meric bundle of helices. A pair of coiled coils may be brought toward each other to form a parallel configuration or an anti-parallel pair configuration. At a same end of the parallel configuration, the coiled coils may not touch each other but the coiled coils may be close enough to form a reaction thereof. In this regard, use of anti-parallel pairs may be considered to be more suitable than parallel pairs.

In various embodiments, the fusion protein may have the amino acid sequence set forth in any one of SEQ ID NOs: 4-5, 11, 14-15.

In a second aspect, a method for detecting a presence or amount of an analyte molecule is provided. The method comprises contacting a fusion protein according to various embodiments of the present invention with the analyte molecule under conditions that allow binding of A to the analyte molecule; and detecting the presence of the analyte molecule by determining a signal produced by the fusion protein:analyte complex.

As used herein, the term "contacting" may refer to reacting or binding. Contacting may be bringing a compound and a target together such that the compound can affect the activity of the target. Contacting may be but is not limited to being performed in a test tube or a petri-dish. Contacting may involve incubation.

The term "detecting" refers to monitoring, determining, or sensing. The term "presence" may refer to the existence or a measureable level of.

The term "under conditions" refers to being subject to a certain set of requirements or parametric control to achieve binding of A to the analyte molecule. For example, the conditions may be but is not limited to temperature and/or length of time. The term "determining" refers to measuring, deriving, or checking. The term "fusion protein:analyte complex" is formed when the fusion protein binds with the analyte, thus forming a complex. As used herein, an "amount" may represent a measurable level.

The terms "fusion protein", "detectable signal", and "binding" are as defined hereinabove.

In various embodiments, the presence or amount of the analyte molecule may be detected in a sample.

In another embodiment, the step of contacting the fusion protein with the analyte molecule may comprise contacting the fusion protein with a sample suspected to contain the analyte molecule.

For example, the step of detecting the presence of the analyte molecule may further include comparing the detected analyte molecule with a control measurement. The "control" measurement may be a positive or negative control measurement. The control measurement serves as a reference or basis against which comparison may be made to the detected measurement.

In various embodiments, the signal produced by the fusion protein may be determined by fluorescence, absorbance, luminescence, enzymatic activity, use of a detectable functional product, use of a selectable phenotype, use of a screenable phenotype that produces an activity due to a phenotypic change. For example, the selectable phenotype may comprise an antibiotic resistance. In an example, the signal produced by the fusion protein may be determined by use of in vivo expression.

The B-C binding and the A-analyte molecule binding are mutually incompatible. In other words, the B-C binding cannot bind to the A-analyte molecule binding.

In various embodiments, the method may be performed in a living cell in vivo or in vitro (ex vivo). The cell may be used for a drug screening assay. For example, the use of the biosensor may be for directed evolution studies in cells (typically bacteria).

In a third aspect, a kit for detecting a presence of an analyte molecule or an amount of analyte is provided. The kit comprises a fusion protein as defined above.

The kit may further comprise a substance for detecting the signal produced by the fusion protein is provided. The fusion protein may be as defined hereinabove.

In order that the invention may be readily understood and put into practical effect, particular embodiments will now be described by way of examples and not limitations, and with reference to the figures.

## EXAMPLES

### Example 1

Anti c-myc Epitope Biosensor

Example 1 describes the development of an anti c-myc epitope biosensor. Apart from a high affinity binding protein, an enzyme capable of providing a convenient read-out (beta-lactamase TEM1 (SEQ ID NO: 1) in this example, but it is noted that any other enzyme may be used) and a protein inhibitor of the said enzyme (beta-lactamase inhibitor protein, or BLIP (SEQ ID NO: 2), in this case) are required. In Example 1, the anti c-myc epitope biosensor was constructed by placing the linker encompassing the c-myc epitope GGSEQKLISEEDGG (SEQ ID NO: 3) between BLIP and TEM1. The anti c-myc epitope biosensor may be BLIP-GGGS-c myc epitope-GGGGSGGGGSGGGGSGGGGG-TEM1 or TEM1-GGGS-c myc epitope-GGGGSGGGGSGGGGSGGGGG-BLIP (SEQ ID NOs: 4 and 5) wherein -GGGS- (SEQ ID NO: 6) and -GGGGSGGGGSGGGGSGGGGG- (SEQ ID NO: 7) are examples of linkers.

The rationale is that while in the absence of anti c-myc antibody the c-myc epitope peptide would be flexible, allowing BLIP-TEM1 binding, the introduction of anti c-myc antibody would rigidify the c-myc epitope and abolish the flexibility required to allow BLIP-TEM1 binding, thereby activating TEM1.

As used herein, the term "rigidify" refers to being rigid, in contrast, to being flexible. The level of rigidness or hardness may be measurable. In this context, rigidness may occur at the backbone of the peptide or protein domain.

The analyte responsiveness of the anti c-myc biosensors reveals strong support for the idea that integrating a read-out providing module such as TEM1, its inhibitor (BLIP) and analyte binding module(s) in such a manner that BLIP-TEM1 binding and binder-analyte binding are mutually incompatible is a viable generic method of generating homogenous one-step biosensors solely using pre-existing well-characterized protein domains. At the same time, the sensitivity obtained with the current first generation biosensors is low. Solutions to improve the sensitivity include placing an analyte binding module outside the BLIP-TEM1 interaction to increase the local concentration of the analyte, which may then subsequently be bound by a second binding module whose binding is incompatible with TEM1-BLIP interaction. The BLIP-TEM1 affinity may be fine-tuned using known mutants of BLIP to maximize signal to noise, lengthening the linker to decrease the background, using high signal fluorescent or chemiluminescent substrates instead of low signal chromogenic substrates and secondary amplification strategies. Additionally, in silico modeling needs to be explored to narrow down the search for the most promising configurations, linker lengths etc.

### Example 2

Detection of anti-HIV p17 epitope antibody

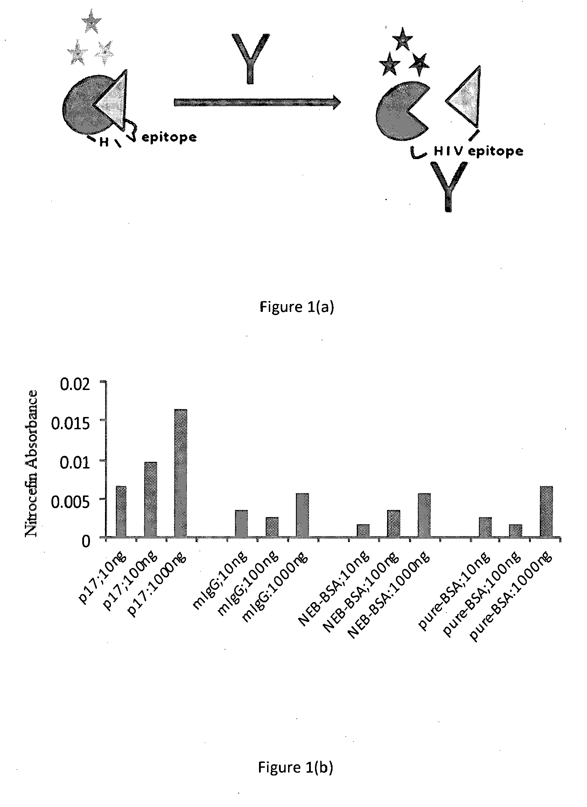

The conserved HIV p17 epitope EKIRLR (SEQ ID NO: 8) was placed as a linker joining the C terminus of TEM1 and the N terminus of BLIP (Figure 1(a)). It was expected that binding of the relevant antibody (mouse monoclonal anti-p17, Clone 32/1.24.89, Zeptometrix) to this epitope would rigidify the epitope and constrain the flexibility required to allow easy Tem1-BLIP binding. Figure 1(b) shows the rate of substrate turnover, as measured by nitrocefin absorbance (y-axis) of the HIV p 17 epitope biosensor in the presence of various analytes.

In Figure 1(b), starting from the left, p17 refers to the anti-EKIRLR mouse monoclonal antibody. The amount of this antibody present in the 25 μl reaction is mentioned (10, 100 or 1000ng). The next 3 histograms show biosensor response to various amounts of mouse whole IgG (mIgG, used as a negative control). The next two sets of three refer to BSA, as a negative control, from different sources, also used at 10, 100 or 1000 ng in a 25 μl reaction. The gradients were normalized by substracting the rate of nitrocefin turnover in the absence of any analyte (background).

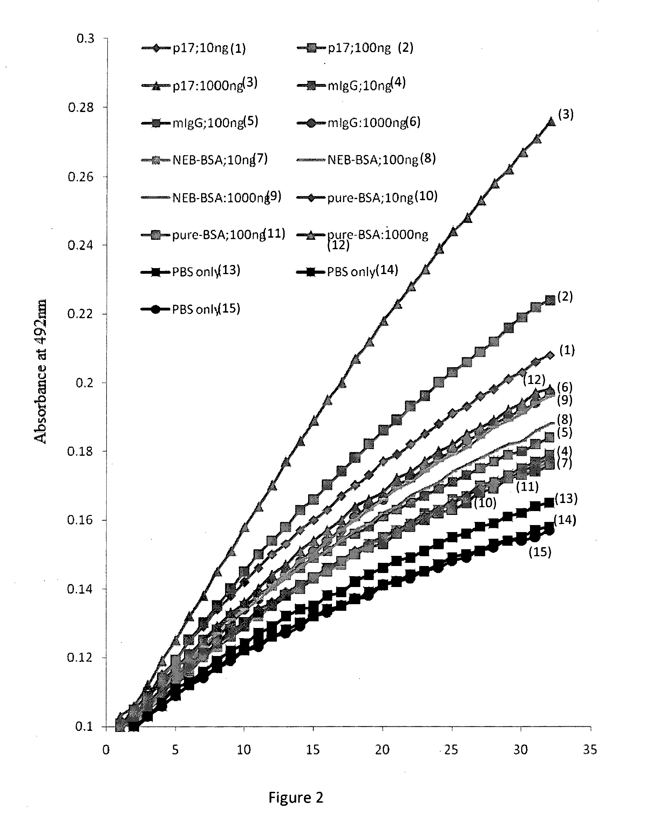

Figure 2 shows OD492 (used to estimate nitrocefin cleavage by TEM1 lactamase, y-axis, read number on x-axis) increase in the presence of various analytes. As in Figure 1, p17 refers to anti-HIV p17 mouse monoclonal antibody, mIgG refers to mouse whole IgG used as a negative control, NEB-BSA and pure-BSA refer to BSA from two different sources used as negative controls, PBS refers to background biosensor activity in buffer in the absence of any analyte. The amounts after the semi-colons refer to the amounts of the analytes present a 25 μl reaction.

Detection of DO1 antibody, and its use in a competitive assay to detect various amounts of a p53 derived peptide

The DO1 antibody binds to the SDLWKLL (SEQ ID NO: 9) linear peptide epitope from p53. This amino acid sequence was placed between TEM1 and BLIP similar to the anti-HIV peptide mentioned above. The biosensor was expressed and tested for responsiveness to DO1 antibody. Detection of free p53 derived peptide in solution was demonstrated by titrating in various amounts of free p53 peptide into the reaction containing DO1 antibody and biosensor. It was expected that the degree of activation of biosensor would drop as the antibody epitope binding sites became blocked by free p53-derived peptide, and the amount of p53 peptide present may be estimated by the extent of the drop in activation. This concept may be extrapolated to free proteins in solution or cell lysates, epitope tags such as myc or HA etc.

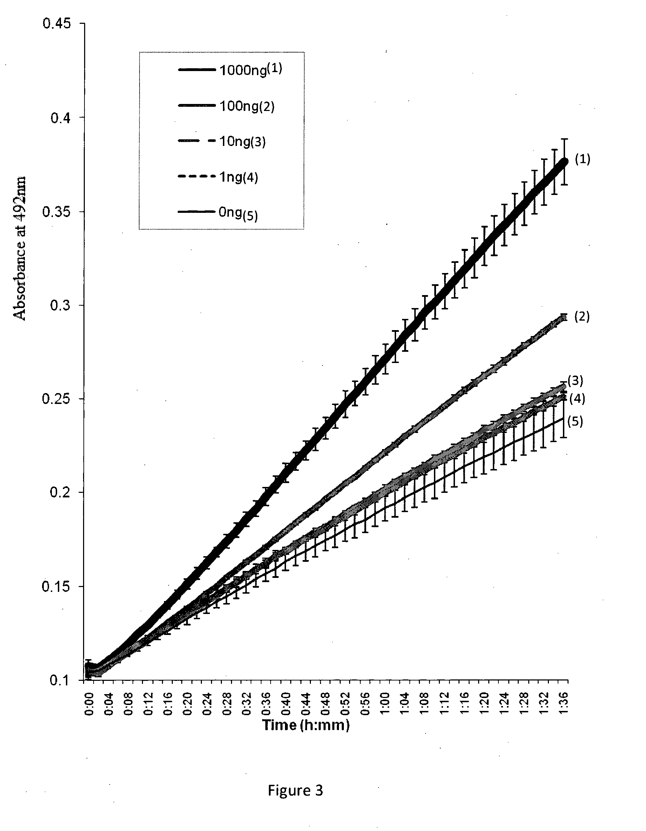

Figure 3 shows increasing amounts of DO1 antibody (0, 1, 10, 100 and 1000ng) were added to biosensor in a 25$\mu$l reaction, and the response monitored by nitrocefin cleavage absorbance at OD 492 nm.

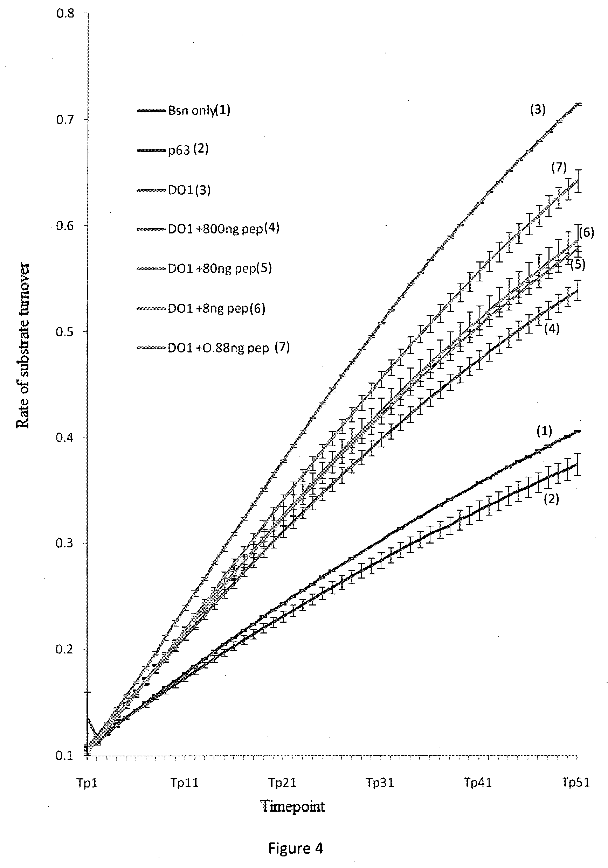

Figure 4 shows various analytes were added to DO1 biosensor and the rate of substrate turnover was monitored (timepoints on x-axis). 1 $\mu$g of p63 antibody (negative control) and DO1 antibody (positive control are shown) along with buffer only control. Various amounts of free DO1 epitope peptide were added into the reaction to demonstrate competitive inhibition of biosensor due to occupation of the epitope binding sites in DO1.

*Example 3*

Detection of Mdm2 ligands nutlin and p53 derived peptide using a Mdm2 biosensor

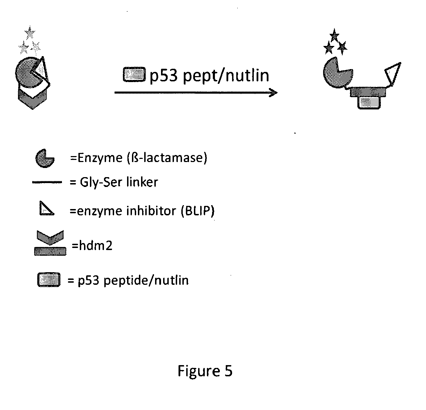

The N terminus of Mdm2 binds and inhibits p53 and is an attractive target for cancer therapy. It is bound by a small molecule, nutlin which is a competitive inhibitor of p53 binding to Mdm2 (SEQ ID NO: 10; UniProtKB accession number Q00987). In addition it is bound by a linear peptide derived from p53. A Mdm2 based sensor was constructed by placing the Mdm2 N terminus between TEM1 and BLIP. It was expected that the Mdm2 N terminus, which is known to have a molten globule structure would be distorted due to the constraint of spanning the distance from the C terminus of BLIP to the N terminus of TEM1. Addition of interactants like nutlin or p53 derived peptide may rigidify the Mdm2 N terminus and drive it to adopt its compact structure, leaving it unable to span the Tem1-BLIP distance, thereby separating some of the TEM1 molecules from BLIP, resulting in increased activity. This concept is schematically illustrated in Figure 5.

In Figure 5, it is shown how rigidification of Mdm2 upon binding its cognate analytes may lead to increased TEM1 lactamase activity.

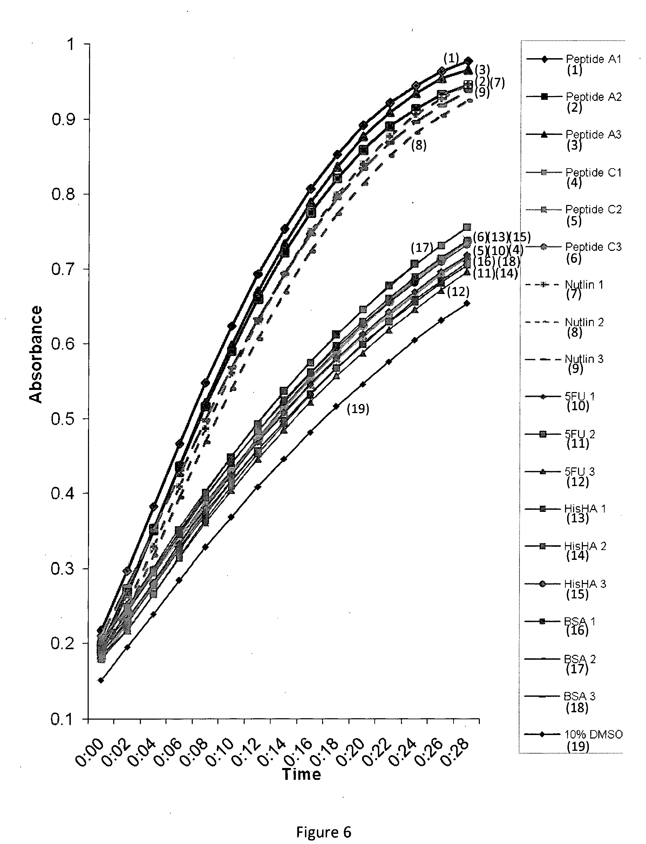

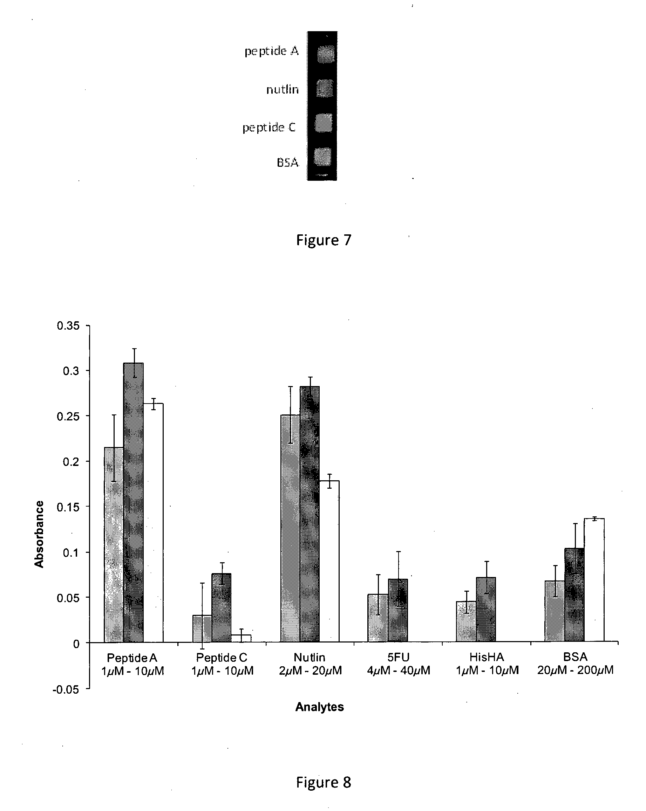

Figure 6 shows the findings wherein various analytes in triplicates (indicated by the suffixed numbers 1-3) were added to Mdm2 biosensor in a 25 $\mu$l reaction. The final concentration of each analyte is 4 $\mu$g/ml. 1$\mu$l of 10% DMSO (solvent used for the peptides and nutlin) was added as a buffer only control. The known interactants nutlin and peptide A (p53 derived Mdm2 binding peptide) show higher rates of substrate turnover than the negative controls peptide C (mutated p53 peptide), HisHA (non-interacting peptide), 5 FU (used as a small molecule control), BSA and no-analyte control. The visual appearance of the reactions is shown in Figure 7.

Figure 8 shows various concentrations of indicated interacting and non-interacting analytes were added to Mdm2 biosensor. The rates of substrate turnover in response as plotted. BSA is at a much lower concentration than the other analytes as its molecular weight is much greater. The mass/volume concentration of BSA is comparable to the other analytes.

*Example 4*

Biosensor function in *in vivo* assay

The DO1 biosensor described in Figures 3-4 (Tem1-SDLWKLL-BLIP) (SEQ ID NO: 11) was expressed in the periplasm of E.coli. Two derivatives with the wild type (WT) SDLWKLL sequence mutated to SGLWKLL and AGLWKLL as denoted in SEQ ID NOs: 12 and 13 respectively were expressed as controls. DO1 scFv was also expressed in these cells and directed to the periplasm using a suitable signal peptide. It was expected that DO1 scFv would bind to and activate the DO1 biosensor. As TEM1 activation leads to resistance to ampicillin and related antibiotics, it was expected a growth advantage would be conferred on the bacteria expressing a biosensor with the correct (SDLWKLL) but not the mutant (SGLWKLL and AGLWKLL) epitope between TEM1 and BLIP (SEQ ID NOs: 14 and 15, respectively). This method may then be used in screening for desirable properties in DO1 scFv, such as binding to a novel epitope, for instance.

Figure 9 shows JM109 cells transformed with expression plasmids encoding indicated biosensor (Kan resistance cassette) and DO1 ScFv (Chlor resistance cassette). Cells were grown to OD ~ 0.5 and plated on ampicillin plates (right panel) or Kan + Chlor plates (left panel). Interaction between DO1 antibody and biosensor with correct epitope (SDLWKL) gives rise to lactamase activity and survival on ampicillin plates (right panel, top row). Reduced or no growth is seen when the epitope is mutated in the SGLWKL and AGLWKL biosensors (right panel, second and third row). Left panel indicates equivalent cell numbers used in assay.

*Example 5*

Conditionally fluorescent peptide biosensor

The biosensor concept described herein does not have to be limited to an enzyme-inhibitor system. The concept was extrapolated to a fluorophore-quencher pair. The fluorophore EDANS and the quencher dabcyl were used. As the fluorophore-quencher do not have significant intrinstic affinity for each other, the fluorophore and quencher were fused each to one member of a coiled-coil pair known to bind each other with high affinity in an antiparallel orientation at positions that place the fluorophore and quencher in close proximity, enabling efficient quenching. The N and C termini of the coils were linked by a peptide recognized by the DO1 antibody, analogous to the usage of this same peptide to link TEM1-BLIP N and C termini in the DO1 biosensor (see Example 2 above). The constraints imposed by having to link N and C termini of the coils should cause the DO1 epitope peptide to adopt a loop orientation, which, as with the enzymatic biosensors, should be disrupted by the rigidification engendered by cognate antibody binding. This may strain the coiled-coil binding, and cause a percentage of the coiled coils to become unbound, increasing fluorophore-quencher distance, enabling fluorescent emission from the fluorophore. This concept is schematically depicted in Figure 10.

Figure 10 shows that in the absence of analyte, the aptamer adopts a flexible loop conformation required to allow coiled-coil binding, thereby keeping the fluorophore close to the quencher. Analyte binding compels the aptamer to adopt a different conformation, straining the coiled coils, leading to their separation and therefore increased fluorescence.

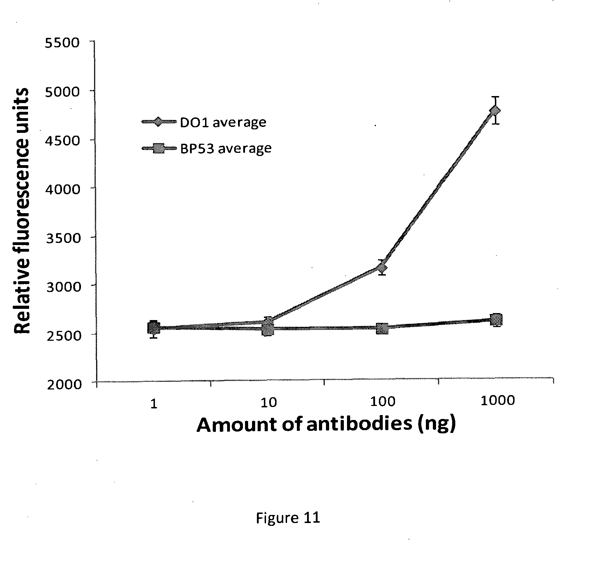

The peptide described above was synthesized chemically and tested. Figure 11 shows the response of the conditionally fluorescent p53 short peptide (with the DO1 antigen peptide SDLWKLL between the antiparallel coiled coils) against specific (DO1) and non-specific (BP53) antibodies. In Figure 1 1, the peptide with a p53 derived DO1 epitope linking the N and C termini of coiled coils carrying a fluorophore and quencher as in Figure 10 were tested with cognate (DO1) and non-cognate (BP53) antibodies in a 25 $\mu$l reaction. The amount of antibody is each reaction is shown on the x-axis. Each data point represents triplicate experiments. DO1 antibody clearly leads to significantly higher fluorescence, as predicted.

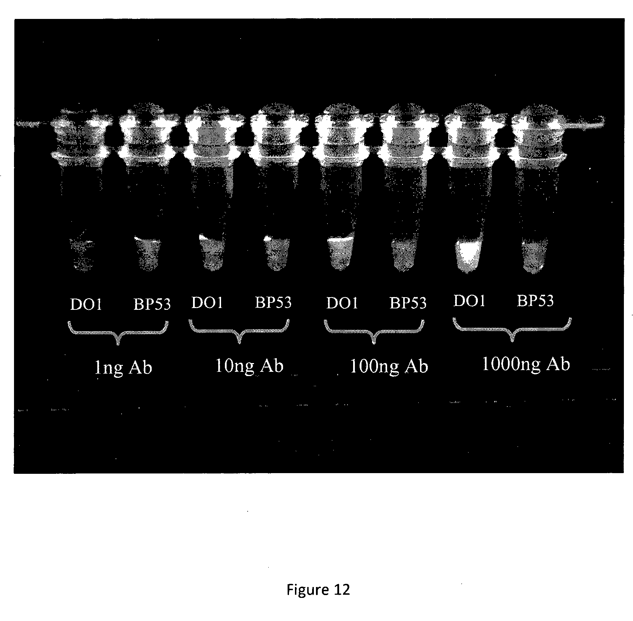

Figure 12 shows samples from Figure 10 being excited and the resulting emission was imaged.

The listing or discussion of a previously published document in this specification should not necessarily be taken as an acknowledgement that the document is part of the state of the art or is common general knowledge.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognise that the invention is also thereby described in terms of any individual member or subgroup of members of the Markush group.

SEQUENCE LISTING

<110> Agency for science, Technology and Research

<120> A FUSION PROTEIN AND METHOD FOR DETECTING A PRESENCE OF AN ANALYTE MOLECULE

<130> P106216

<150> SG Patent Application No. 201102087-2
<151> 2011-03-23

<160> 15

<170> PatentIn version 3.5

<210> 1
<211> 286
<212> PRT
<213> artificial

<220>
<223> Beta lactamase TEM-1

<400> 1

```
Met Ser Ile Gln His Phe Arg Val Ala Leu Ile Pro Phe Phe Ala Ala
1           5               10              15

Phe Cys Leu Pro Val Phe Ala His Pro Glu Thr Leu Val Lys Val Lys
            20              25              30

Asp Ala Glu Asp Gln Leu Gly Ala Arg Val Gly Tyr Ile Glu Leu Asp
        35              40              45

Leu Asn Ser Gly Lys Ile Leu Glu Ser Phe Arg Pro Glu Glu Arg Phe
    50              55              60

Pro Met Met Ser Thr Phe Lys Val Leu Leu Cys Gly Ala Val Leu Ser
65              70              75              80

Arg Val Asp Ala Gly Gln Glu Gln Leu Gly Arg Arg Ile His Tyr Ser
            85              90              95

Gln Asn Asp Leu Val Glu Tyr Ser Pro Val Thr Glu Lys His Leu Thr
            100             105             110

Asp Gly Met Thr Val Arg Glu Leu Cys Ser Ala Ala Ile Thr Met Ser
        115             120             125

Asp Asn Thr Ala Ala Asn Leu Leu Leu Thr Thr Ile Gly Gly Pro Lys
        130             135             140

Glu Leu Thr Ala Phe Leu His Asn Met Gly Asp His Val Thr Arg Leu
145             150             155             160

Asp Arg Trp Glu Pro Glu Leu Asn Glu Ala Ile Pro Asn Asp Glu Arg
                165             170             175
```

```
Asp Thr Thr Met Pro Ala Ala Met Ala Thr Thr Leu Arg Lys Leu Leu
            180                 185                 190

Thr Gly Glu Leu Leu Thr Leu Ala Ser Arg Gln Gln Leu Ile Asp Trp
            195                 200                 205

Met Glu Ala Asp Lys Val Ala Gly Pro Leu Leu Arg Ser Ala Leu Pro
    210                 215                 220

Ala Gly Trp Phe Ile Ala Asp Lys Ser Gly Ala Gly Glu Arg Gly Ser
225             230                 235                     240

Arg Gly Ile Ile Ala Ala Leu Gly Pro Asp Gly Lys Pro Ser Arg Ile
            245                 250                     255

Val Val Ile Tyr Thr Thr Gly Ser Gln Ala Thr Met Asp Glu Arg Asn
            260                 265                 270

Arg Gln Ile Ala Glu Ile Gly Ala Ser Leu Ile Lys His Trp
            275                 280                 285
```

<210> 2
<211> 1743
<212> PRT
<213> artificial

<220>
<223> Beta lactamase inhibitor protein (BLIP) D49A

<400> 2

```
Met Lys Gln Asn Lys Leu Leu Gln Arg Gly Ala Tyr Phe Asn Asp Lys
1               5                   10                  15

Asn Ile Leu Ile Asp Asp Phe Asp Lys Arg Tyr Asn Asp Tyr Asp Phe
            20                  25                  30

Val Glu Phe Phe Thr Gly Ile Ser Asn Ser Thr Phe Gly Leu Lys Ser
            35                  40                  45

Asp Gly Asn Leu Tyr Ala Cys Gly Asp Asn Thr Gly Phe Gln Leu Gly
    50                  55                  60

Leu Gly Lys Asp Ser Ser Glu Arg Arg Met Phe Ser Lys Val Lys Ile
65                  70                  75                  80

Asp Asn Val Lys Tyr Val Ser Cys Gly Ser Lys His Ser Val Ala Val
            85                  90                  95

Thr Lys Asp Gly Phe Ala Tyr Gly Ala Gly Thr Ser Asn Val Gly Gln
            100                 105                 110
```

Leu Gly Val Ile Glu Ser Thr Val Tyr Tyr Glu Phe Thr Lys Leu Pro
115                     120                     125

Ile Asp Asp Val Lys Thr Val Ala Cys Gly Tyr Asp Phe Thr Phe Val
    130             135             140

Leu Lys Asn Asp Gly Thr Leu Tyr Ser Ala Gly Leu Asn Ser Ser Gly
145             150             155             160

Gln Leu Gly Leu Gly Asp Thr Asn Asn Arg Val Thr Phe Thr Lys Val
            165             170             175

Asn Ile Asp Ser Val Lys Asp Val Val Thr Tyr Asn Gln Ser Val Phe
            180             185             190

Ile Ile Lys Met Asp Gly Thr Ala His Ala Cys Gly Leu Asn Ser Asn
        195             200             205

Gly Gln Leu Gly Ile Asn Ser Thr Leu Asn Lys Ser Val Phe Asn Lys
210             215             220

Ile Glu Gly Met Asp Asn Val Lys Gln Ile Ala Cys Gly Ser Ser His
225             230             235             240

Thr Ile Leu Ile Lys Asn Asp Gly Thr Met Tyr Thr Thr Gly Ser Asn
            245             250             255

Gly Tyr Gly Gln Leu Gly Thr Gly Asn Asn Asn Ser Ile Val Phe
            260             265             270

Thr Leu Ser Ser Ile Asn Asn Val Lys Tyr Ala Ser Cys Gly Asn Asn
        275             280             285

His Thr Met Ile Leu Lys Tyr Asp Asn Thr Leu Phe Ser Thr Gly Gln
    290             295             300

Asn Asn Tyr Gly Gln Leu Ala Asn Ala Asn Lys Asp Val Ala Ser Arg
305             310             315             320

Asn Thr Phe Val Lys Val Asn Val Glu Asn Ile Lys Asp Ile Lys Cys
            325             330             335

Gly Ser Gln Phe Asn Phe Leu Ile Asn Gly Ser Lys Glu Ile Phe Val
        340             345             350

Ser Gly Cys Asn Leu Ala Gly Gln Leu Gly Ser Phe Phe His Thr Thr
        355             360             365

Phe Leu Tyr Glu Phe Ser Lys Val Gln Ser Ser Asn Leu Asp Asn Tyr
    370             375             380

```
Ser Gly Leu Leu Val Asn Asp Asp Tyr Leu Tyr Val Thr Lys Asp Asn
385                     390                 395                 400

Ser Glu Phe Leu Asn Val Lys Leu Ser Asp Asn Phe Gln Asp Tyr Lys
                405             410             415

Lys Ile Glu Leu Thr Asp Asn Asn Met Phe Ile Val Met Asn Asp Gly
                420             425             430

Thr Leu Tyr Ala Cys Gly Leu Asn Asn Tyr Gly Gln Leu Gly Leu Gly
            435             440             445

Asp Thr Val Asn Arg Ser Val Met Thr Lys Val Asp Ile Asp Asn Val
    450             455             460

Leu Asp Ile Lys Gly Asn Gly Asn Ser Thr Phe Val Leu Lys Asn Asn
465             470             475             480

Gly Thr Leu Tyr Ser Cys Gly Tyr Asn Ser Ser Gly Ile Leu Gly Leu
            485             490             495

Lys Asp Asn Thr Asn Arg Asn Ile Phe Thr Lys Ile Glu Ile Glu Asn
            500             505             510

Ile Lys Glu Phe Cys Val Glu Ser Asn Tyr Ile Val Ala Leu Asn His
        515             520             525

Ser Lys Glu Leu Tyr Gly Trp Gly Asn Gln Ser Tyr Ile Val Tyr Gly
    530             535             540

Asp Asn Arg Asn Tyr Pro Tyr Lys Asp Thr Arg Val Ser Asn Val Glu
545             550             555             560

Lys Ile Ala Thr Trp Ser Asp Thr Leu Tyr Ile Leu Asp Ser Thr Gly
            565             570             575

Ala Thr Lys Thr Ile Gly Tyr Ser Tyr Asn Gly Ser Gly Gly Tyr Pro
            580             585             590

Ala Pro Ser Ser Ser Ser Thr Tyr Arg Glu Gly Gly Tyr Ile Asn Lys
        595             600             605

Asn Thr Ser Tyr Arg Thr Leu Glu Phe Tyr Asn Thr Ser Lys Thr Lys
    610             615             620

Leu Val Asn Leu Phe Ala Phe Tyr Asn Gly Cys Val Phe Val Asp Glu
625             630             635             640

Asn Gly Leu Ala Tyr Cys Ile Gly Glu Asn Asn Ile Asn Phe Arg Gly
            645             650             655
```

13

```
Gly Ser Thr Thr Asn Glu Asn Asn Ser Leu Arg Phe Ile Asn Asn Ser
            660                     665                 670

Gly Val Tyr Tyr Thr Asn Thr Asp Gly Thr Asp Tyr Thr Cys Tyr Gln
        675                 680                 685

Trp Thr Tyr Lys Leu Ile Arg Cys Ser Ile Phe Asp Ser Pro Gln Asn
    690                 695                 700

Ile Ile Gly Asn Ser Lys Asn Ile Leu Tyr Leu Ser Lys Asn Asn Ser
705                 710                 715                 720

Thr Phe Lys Cys Thr Gly Asn Cys Ile Thr Tyr Gly Ile Asn Ser Gln
            725                 730                 735

Asn Trp Tyr Ser Tyr Phe Ser Asp Ser Ser Asn Gly Ala Ile Ala Leu
            740                 745                 750

Gly Asn Glu Phe Ile Leu Lys Asn Tyr Ser Gly Glu Cys Leu Leu Lys
        755                 760                 765

Gly Tyr Gly Lys Ala Thr Asn Gly Glu Phe Gly Asn Ser Thr Asn Ile
    770                 775                 780

Ser Ser Ile Ser Asn Tyr Asp Thr Gly Leu Lys Asp Ile Lys Asp Ile
785                 790                 795                 800

Ile Val Lys Asn Asn Thr Val Val Val Asp Lys Asn Asn Asn Ile
            805                 810                 815

Tyr Val Thr Gly Ala Asn Gln Phe Asn Lys Leu Gly Ile Gly Glu Tyr
            820                 825                 830

Asn Asn Gln Pro Ile Arg Lys Phe Thr Asn Ile Thr Glu Gln Ser Asn
        835                 840                 845

Ser Phe Ile Phe Met Asp Asp Ile Lys Glu Ile Thr Thr Ser Arg Asn
    850                 855                 860

Thr Met Phe Ile Val Lys Asn Asp Gly Thr Ala Tyr Ala Thr Gly Asn
865                 870                 875                 880

Asn Ser Ser Gly Gln Leu Gly Leu Gly Asp Thr Ile Asn Arg Asn Lys
            885                 890                 895

Phe Thr Gln Ile Asn Leu Asp Asn Ile Lys Lys Ile Ser Thr Ser Ile
            900                 905                 910

Asp Gly Asn Thr Thr Phe Ala Ile Arg Asn Asp Gly Thr Leu Tyr Ser
            915                 920                 925
```

14

Thr Gly Leu Asn Thr Lys Gly Gln Leu Gly Leu Gly Asp Ile Val Asn
930 935 940

Arg Asn Thr Phe Thr Lys Val Asn Ile Gln Asn Val Arg Asp Val Val
945 950 955 960

Leu Gly Thr Thr His Ser His Ala Ile Lys Asp Asp Asn Thr Leu Tyr
965 970 975

Ser Cys Gly Glu Asn Thr His Gly Gln Leu Gly Leu Gly Ser Glu Ser
980 985 990

Asn His Pro Asp Val Leu Thr Phe Thr Val Asn Asn Ile Thr Asn Val
995 1000 1005

Arg Asp Val Tyr Cys Ser Asp Thr Thr Thr Phe Ile Val Lys Asp
1010 1015 1020

Thr Asn Ile Ala Tyr Cys Cys Gly Tyr Asn Asn Asn Ser Gln Leu
1025 1030 1035

Gly Met Gly Asn Thr Thr Asp Gln Tyr Ser Phe Ile Lys Cys Met
1040 1045 1050

Glu Asn Val Lys Glu Val Ile Pro Asn Glu Ile Asn Thr Tyr Ile
1055 1060 1065

Ile Thr Ile Tyr Asn Thr Ala Tyr Ser Thr Gly Leu Asn Thr Asp
1070 1075 1080

Tyr Cys Leu Gly Leu Asn Ser Asn Ser Asn Gln Ser Ser Phe Ser
1085 1090 1095

Glu Ile Pro Ile Ser Asn Val Val Lys Val Ala Pro Asn Arg Asn
1100 1105 1110

Asn Ala Val Leu Leu Leu Thr Ser Glu Gly Asp Val Tyr Thr Ala
1115 1120 1125

Gly Lys Cys Ser Asn Gly Ser Gly Thr Gly Ser Glu Thr Pro Glu
1130 1135 1140

Lys Ile Lys Lys Ile Ala Ser Lys Ala Lys Asp Ile Gly Met Asn
1145 1150 1155

Tyr Arg Cys Gly His Tyr Val Ser Asp Asn Gly Asp Leu Tyr Gly
1160 1165 1170

Thr Gly Phe Asn Asp Cys Gly Gln Leu Gly Val Gly Asn Val Thr
1175 1180 1185

```
Lys Arg  Asp Thr Phe Ile Lys  Thr Asn Thr Arg Val  Lys Lys Ile
      1190                 1195                 1200

Leu Pro  Leu Glu Tyr Ala Asn  Ile Ala Ile Lys Asp  Thr Asn Asp
    1205                 1210                 1215

Ile Tyr  Ile Cys Gly Leu Asn  Asn Tyr Gly Gln Leu  Gly Val Gly
    1220                 1225                 1230

Asn Arg  Tyr Asp Ser Arg Asn  Asn Asp Asn Arg Ile  Phe Asn Tyr
    1235                 1240                 1245

Lys His  Met Asn Phe Val Met  Gly Asp Leu Thr Ser  Ile Lys Asn
    1250                 1255                 1260

Arg His  Asn Phe Ile Leu Leu  Asn Asn Lys Ile Val  Ile Pro Thr
    1265                 1270                 1275

Thr Lys  Asp Ile Asp Tyr Gly  Leu Val Leu Gly Asn  Leu Tyr Lys
    1280                 1285                 1290

Gly Asp  Leu Tyr Thr Glu Leu  Pro Tyr Glu Asp Ile  Lys Glu Val
    1295                 1300                 1305

Ser Ile  Ser Lys Thr His Ile  Ile Ile Leu Leu Asn  Asp Gly Thr
    1310                 1315                 1320

Met Tyr  Gly Cys Gly Thr Asn  Tyr His Gly Glu Leu  Leu Gln Asp
    1325                 1330                 1335

Leu Ser  Ile Asn Gln Val Asp  Glu Phe Val Gln Ile  Asn Val Ser
    1340                 1345                 1350

Asp Val  Lys His Val Ser Cys  Gly Asp Asn Phe Thr  Tyr Phe Ile
    1355                 1360                 1365

Lys Ser  Asp Asp Ser Leu Trp  Ser Ile Gly Lys Asn  Ser Glu Tyr
    1370                 1375                 1380

Gln Leu  Gly Ile Gly His Asn  Asn Pro Val Thr Glu  Leu Gln Arg
    1385                 1390                 1395

Ile Thr  Thr Ile Ser Ser Cys  Lys Glu Val His Cys  Gly Lys Asn
    1400                 1405                 1410

Tyr Thr  Leu Val Val Thr Thr  Ser Asn Glu Leu Phe  Val Gln Gly
    1415                 1420                 1425

Tyr Asn  Asp Lys Gly Ala Leu  Gly Leu Gly Ser Asp  Ser Glu Asn
    1430                 1435                 1440
```

```
Thr Ile  Ile Lys Phe Phe Thr  Lys Ala Leu Thr Asp  Ile Arg Glu
    1445                  1450                  1455

Ile Lys  Ser Tyr Gly Ser Asp  His Ile Leu Val Leu  Lys Asn Asp
    1460              1465                  1470

Asn Ser  Val Trp Val Thr Gly  Lys Asn Arg Asp Val  Tyr Lys Ile
    1475              1480                  1485

Glu Gln  Pro Val Glu Phe Leu  Lys Glu Phe Thr Ile  Val Pro Ile
    1490              1495                  1500

Ser Glu  Asp Val Asn Thr Val  Lys Asp Val Leu Ala  Thr Asp Asn
    1505              1510                  1515

Thr Leu  Tyr Ile Ile Ser Glu  Val Gly Thr Thr Asn  Ala Ala Ile
    1520              1525                  1530

Glu Ile  Thr Glu Lys Ser Ile  Ser Ser Ile Lys Ile  Lys Ile Gln
    1535              1540                  1545

Asp Pro  Asn Lys Asp Ile Ser  Arg Ile Glu Met Leu  Ile Asn Gly
    1550              1555                  1560

Glu Ser  Val Lys Ser Val Ser  Asp Leu Ile Thr Glu  Lys Ile Ser
    1565              1570                  1575

Phe Glu  Val Pro Pro Asp Lys  Ile Lys Ile Gly Glu  Asn Lys Ile
    1580              1585                  1590

Leu Phe  Arg Ala Tyr Cys Lys  Gly Asp Asp Leu Tyr  Ala Ser Leu
    1595              1600                  1605

Phe Ile  Phe Lys Glu Ser Thr  Gly Asn Ser Ile Ile  Lys Asp Ser
    1610              1615                  1620

Tyr Val  Met Ile Gly Asn Arg  Met Tyr Lys Val Val  Asn Thr Thr
    1625              1630                  1635

Ser Asn  Glu Gln Asp Ile Thr  Ile Thr Leu Asp Arg  Gly Leu Glu
    1640              1645                  1650

Glu Asp  Leu Asn Leu Gly Asp  Pro Ile Tyr Gln Leu  Ile Asn Lys
    1655              1660                  1665

Thr Lys  Val Gln Val Lys Ile  Asn Lys Ser Asp Leu  Phe Lys Asp
    1670              1675                  1680

Met Lys  Leu Val Glu Ile Lys  Lys Ser Asp Ser Ser  Tyr Gln Glu
    1685              1690                  1695
```

17

```
Ile Tyr Glu Leu Glu Glu Ala Asn Ile Lys Ser Ala Gln Pro Lys
        1700                1705                1710

Ile Ile Val Glu Lys Gly Asp Lys Trp Thr Ala Ile Lys Arg Pro
        1715                1720                1725

Ser Met Ile Phe Arg Tyr Asp Ala Glu Asn Asn Glu Pro Gln Ala
        1730                1735                1740
```

<210> 3
<211> 14
<212> PRT
<213> Homo sapiens

<400> 3

```
Gly Gly Ser Glu Gln Lys Leu Ile Ser Glu Glu Asp Gly Gly
1               5                   10
```

<210> 4
<211> 2067
<212> PRT
<213> artificial

<220>
<223> BLIP- GGSEQKLISEEDGG-Tem1 (BLIP-c myc-TEM1)

<400> 4
```

```
Met Lys Gln Asn Lys Leu Leu Gln Arg Gly Ala Tyr Phe Asn Asp Lys
1               5                   10                  15

Asn Ile Leu Ile Asp Asp Phe Asp Lys Arg Tyr Asn Asp Tyr Asp Phe
            20                  25                  30

Val Glu Phe Phe Thr Gly Ile Ser Asn Ser Thr Phe Gly Leu Lys Ser
        35                  40                  45

Asp Gly Asn Leu Tyr Ala Cys Gly Asp Asn Thr Gly Phe Gln Leu Gly
    50                  55                  60

Leu Gly Lys Asp Ser Ser Glu Arg Arg Met Phe Ser Lys Val Lys Ile
65                  70                  75                  80

Asp Asn Val Lys Tyr Val Ser Cys Gly Ser Lys His Ser Val Ala Val
                85                  90                  95

Thr Lys Asp Gly Phe Ala Tyr Gly Ala Gly Thr Ser Asn Val Gly Gln
            100                 105                 110

Leu Gly Val Ile Glu Ser Thr Val Tyr Tyr Glu Phe Thr Lys Leu Pro
            115                 120                 125

Ile Asp Asp Val Lys Thr Val Ala Cys Gly Tyr Asp Phe Thr Phe Val
    130                 135                 140
```

Leu Lys Asn Asp Gly Thr Leu Tyr Ser Ala Gly Leu Asn Ser Ser Gly
145               150               155                   160

Gln Leu Gly Leu Gly Asp Thr Asn Asn Arg Val Thr Phe Thr Lys Val
                165               170                   175

Asn Ile Asp Ser Val Lys Asp Val Val Thr Tyr Asn Gln Ser Val Phe
                180               185               190

Ile Ile Lys Met Asp Gly Thr Ala His Ala Cys Gly Leu Asn Ser Asn
            195               200               205

Gly Gln Leu Gly Ile Asn Ser Thr Leu Asn Lys Ser Val Phe Asn Lys
    210               215               220

Ile Glu Gly Met Asp Asn Val Lys Gln Ile Ala Cys Gly Ser Ser His
225               230               235               240

Thr Ile Leu Ile Lys Asn Asp Gly Thr Met Tyr Thr Thr Gly Ser Asn
            245               250               255

Gly Tyr Gly Gln Leu Gly Thr Gly Asn Asn Asn Asn Ser Ile Val Phe
            260               265               270

Thr Leu Ser Ser Ile Asn Asn Val Lys Tyr Ala Ser Cys Gly Asn Asn
        275               280               285

His Thr Met Ile Leu Lys Tyr Asp Asn Thr Leu Phe Ser Thr Gly Gln
    290               295               300

Asn Asn Tyr Gly Gln Leu Ala Asn Ala Asn Lys Asp Val Ala Ser Arg
305               310               315               320

Asn Thr Phe Val Lys Val Asn Val Glu Asn Ile Lys Asp Ile Lys Cys
            325               330               335

Gly Ser Gln Phe Asn Phe Leu Ile Asn Gly Ser Lys Glu Ile Phe Val
            340               345               350

Ser Gly Cys Asn Leu Ala Gly Gln Leu Gly Ser Phe Phe His Thr Thr
            355               360               365

Phe Leu Tyr Glu Phe Ser Lys Val Gln Ser Ser Asn Leu Asp Asn Tyr
    370               375               380

Ser Gly Leu Leu Val Asn Asp Asp Tyr Leu Tyr Val Thr Lys Asp Asn
385               390               395               400

Ser Glu Phe Leu Asn Val Lys Leu Ser Asp Asn Phe Gln Asp Tyr Lys
            405               410               415

20

```
Lys Ile Glu Leu Thr Asp Asn Asn Met Phe Ile Val Met Asn Asp Gly
          420                 425                 430

Thr Leu Tyr Ala Cys Gly Leu Asn Asn Tyr Gly Gln Leu Gly Leu Gly
          435                 440                 445

Asp Thr Val Asn Arg Ser Val Met Thr Lys Val Asp Ile Asp Asn Val
          450                 455                 460

Leu Asp Ile Lys Gly Asn Gly Asn Ser Thr Phe Val Leu Lys Asn Asn
465                 470                 475                 480

Gly Thr Leu Tyr Ser Cys Gly Tyr Asn Ser Ser Gly Ile Leu Gly Leu
              485                 490                 495

Lys Asp Asn Thr Asn Arg Asn Ile Phe Thr Lys Ile Glu Ile Glu Asn
          500                 505                 510

Ile Lys Glu Phe Cys Val Glu Ser Asn Tyr Ile Val Ala Leu Asn His
          515                 520                 525

Ser Lys Glu Leu Tyr Gly Trp Gly Asn Gln Ser Tyr Ile Val Tyr Gly
          530                 535                 540

Asp Asn Arg Asn Tyr Pro Tyr Lys Asp Thr Arg Val Ser Asn Val Glu
545                 550                 555                 560

Lys Ile Ala Thr Trp Ser Asp Thr Leu Tyr Ile Leu Asp Ser Thr Gly
              565                 570                 575

Ala Thr Lys Thr Ile Gly Tyr Ser Tyr Asn Gly Ser Gly Gly Tyr Pro
              580                 585                 590

Ala Pro Ser Ser Ser Ser Thr Tyr Arg Glu Gly Gly Tyr Ile Asn Lys
          595                 600                 605

Asn Thr Ser Tyr Arg Thr Leu Glu Phe Tyr Asn Thr Ser Lys Thr Lys
610                 615                 620

Leu Val Asn Leu Phe Ala Phe Tyr Asn Gly Cys Val Phe Val Asp Glu
625                 630                 635                 640

Asn Gly Leu Ala Tyr Cys Ile Gly Glu Asn Asn Ile Asn Phe Arg Gly
              645                 650                 655

Gly Ser Thr Thr Asn Glu Asn Asn Ser Leu Arg Phe Ile Asn Asn Ser
          660                 665                 670

Gly Val Tyr Tyr Thr Asn Thr Asp Gly Thr Asp Tyr Thr Cys Tyr Gln
          675                 680                 685
```

21

Trp Thr Tyr Lys Leu Ile Arg Cys Ser Ile Phe Asp Ser Pro Gln Asn
690                     695             700

Ile Ile Gly Asn Ser Lys Asn Ile Leu Tyr Leu Ser Lys Asn Asn Ser
705             710             715                 720

Thr Phe Lys Cys Thr Gly Asn Cys Ile Thr Tyr Gly Ile Asn Ser Gln
                725             730                 735

Asn Trp Tyr Ser Tyr Phe Ser Asp Ser Ser Asn Gly Ala Ile Ala Leu
            740             745             750

Gly Asn Glu Phe Ile Leu Lys Asn Tyr Ser Gly Glu Cys Leu Leu Lys
        755             760             765

Gly Tyr Gly Lys Ala Thr Asn Gly Glu Phe Gly Asn Ser Thr Asn Ile
        770             775             780

Ser Ser Ile Ser Asn Tyr Asp Thr Gly Leu Lys Asp Ile Lys Asp Ile
785             790             795                 800

Ile Val Lys Asn Asn Thr Val Val Val Asp Lys Asn Asn Asn Ile
                805             810             815

Tyr Val Thr Gly Ala Asn Gln Phe Asn Lys Leu Gly Ile Gly Glu Tyr
            820             825             830

Asn Asn Gln Pro Ile Arg Lys Phe Thr Asn Ile Thr Glu Gln Ser Asn
        835             840             845

Ser Phe Ile Phe Met Asp Asp Ile Lys Glu Ile Thr Thr Ser Arg Asn
        850             855             860

Thr Met Phe Ile Val Lys Asn Asp Gly Thr Ala Tyr Ala Thr Gly Asn
865             870             875                 880

Asn Ser Ser Gly Gln Leu Gly Leu Gly Asp Thr Ile Asn Arg Asn Lys
            885             890             895

Phe Thr Gln Ile Asn Leu Asp Asn Ile Lys Lys Ile Ser Thr Ser Ile
        900             905             910

Asp Gly Asn Thr Thr Phe Ala Ile Arg Asn Asp Gly Thr Leu Tyr Ser
        915             920             925

Thr Gly Leu Asn Thr Lys Gly Gln Leu Gly Leu Gly Asp Ile Val Asn
        930             935             940

Arg Asn Thr Phe Thr Lys Val Asn Ile Gln Asn Val Arg Asp Val Val
945             950             955             960

22

Leu Gly Thr Thr His Ser His Ala Ile Lys Asp Asp Asn Thr Leu Tyr
965 970 975

Ser Cys Gly Glu Asn Thr His Gly Gln Leu Gly Leu Gly Ser Glu Ser
980 985 990

Asn His Pro Asp Val Leu Thr Phe Thr Val Asn Asn Ile Thr Asn Val
995 1000 1005

Arg Asp Val Tyr Cys Ser Asp Thr Thr Thr Phe Ile Val Lys Asp
1010 1015 1020

Thr Asn Ile Ala Tyr Cys Cys Gly Tyr Asn Asn Asn Ser Gln Leu
1025 1030 1035

Gly Met Gly Asn Thr Thr Asp Gln Tyr Ser Phe Ile Lys Cys Met
1040 1045 1050

Glu Asn Val Lys Glu Val Ile Pro Asn Glu Ile Asn Thr Tyr Ile
1055 1060 1065

Ile Thr Ile Tyr Asn Thr Ala Tyr Ser Thr Gly Leu Asn Thr Asp
1070 1075 1080

Tyr Cys Leu Gly Leu Asn Ser Asn Ser Asn Gln Ser Ser Phe Ser
1085 1090 1095

Glu Ile Pro Ile Ser Asn Val Val Lys Val Ala Pro Asn Arg Asn
1100 1105 1110

Asn Ala Val Leu Leu Leu Thr Ser Glu Gly Asp Val Tyr Thr Ala
1115 1120 1125

Gly Lys Cys Ser Asn Gly Ser Gly Thr Gly Ser Glu Thr Pro Glu
1130 1135 1140

Lys Ile Lys Lys Ile Ala Ser Lys Ala Lys Asp Ile Gly Met Asn
1145 1150 1155

Tyr Arg Cys Gly His Tyr Val Ser Asp Asn Gly Asp Leu Tyr Gly
1160 1165 1170

Thr Gly Phe Asn Asp Cys Gly Gln Leu Gly Val Gly Asn Val Thr
1175 1180 1185

Lys Arg Asp Thr Phe Ile Lys Thr Asn Thr Arg Val Lys Lys Ile
1190 1195 1200

Leu Pro Leu Glu Tyr Ala Asn Ile Ala Ile Lys Asp Thr Asn Asp
1205 1210 1215

Ile Tyr Ile Cys Gly Leu Asn Asn Tyr Gly Gln Leu Gly Val Gly
1220 1225 1230

Asn Arg Tyr Asp Ser Arg Asn Asn Asp Asn Arg Ile Phe Asn Tyr
1235 1240 1245

Lys His Met Asn Phe Val Met Gly Asp Leu Thr Ser Ile Lys Asn
1250 1255 1260

Arg His Asn Phe Ile Leu Leu Asn Asn Lys Ile Val Ile Pro Thr
1265 1270 1275

Thr Lys Asp Ile Asp Tyr Gly Leu Val Leu Gly Asn Leu Tyr Lys
1280 1285 1290

Gly Asp Leu Tyr Thr Glu Leu Pro Tyr Glu Asp Ile Lys Glu Val
1295 1300 1305

Ser Ile Ser Lys Thr His Ile Ile Ile Leu Leu Asn Asp Gly Thr
1310 1315 1320

Met Tyr Gly Cys Gly Thr Asn Tyr His Gly Glu Leu Leu Gln Asp
1325 1330 1335

Leu Ser Ile Asn Gln Val Asp Glu Phe Val Gln Ile Asn Val Ser
1340 1345 1350

Asp Val Lys His Val Ser Cys Gly Asp Asn Phe Thr Tyr Phe Ile
1355 1360 1365

Lys Ser Asp Asp Ser Leu Trp Ser Ile Gly Lys Asn Ser Glu Tyr
1370 1375 1380

Gln Leu Gly Ile Gly His Asn Asn Pro Val Thr Glu Leu Gln Arg
1385 1390 1395

Ile Thr Thr Ile Ser Ser Cys Lys Glu Val His Cys Gly Lys Asn
1400 1405 1410

Tyr Thr Leu Val Val Thr Thr Ser Asn Glu Leu Phe Val Gln Gly
1415 1420 1425

Tyr Asn Asp Lys Gly Ala Leu Gly Leu Gly Ser Asp Ser Glu Asn
1430 1435 1440

Thr Ile Ile Lys Phe Phe Thr Lys Ala Leu Thr Asp Ile Arg Glu
1445 1450 1455

Ile Lys Ser Tyr Gly Ser Asp His Ile Leu Val Leu Lys Asn Asp
1460 1465 1470

24

Asn Ser Val Trp Val Thr Gly Lys Asn Arg Asp Val Tyr Lys Ile
1475            1480            1485

Glu Gln Pro Val Glu Phe Leu Lys Glu Phe Thr Ile Val Pro Ile
1490            1495            1500

Ser Glu Asp Val Asn Thr Val Lys Asp Val Leu Ala Thr Asp Asn
1505            1510            1515

Thr Leu Tyr Ile Ile Ser Glu Val Gly Thr Thr Asn Ala Ala Ile
1520            1525            1530

Glu Ile Thr Glu Lys Ser Ile Ser Ser Ile Lys Ile Lys Ile Gln
1535            1540            1545

Asp Pro Asn Lys Asp Ile Ser Arg Ile Glu Met Leu Ile Asn Gly
1550            1555            1560

Glu Ser Val Lys Ser Val Ser Asp Leu Ile Thr Glu Lys Ile Ser
1565            1570            1575

Phe Glu Val Pro Pro Asp Lys Ile Lys Ile Gly Glu Asn Lys Ile
1580            1585            1590

Leu Phe Arg Ala Tyr Cys Lys Gly Asp Asp Leu Tyr Ala Ser Leu
1595            1600            1605

Phe Ile Phe Lys Glu Ser Thr Gly Asn Ser Ile Ile Lys Asp Ser
1610            1615            1620

Tyr Val Met Ile Gly Asn Arg Met Tyr Lys Val Val Asn Thr Thr
1625            1630            1635

Ser Asn Glu Gln Asp Ile Thr Ile Thr Leu Asp Arg Gly Leu Glu
1640            1645            1650

Glu Asp Leu Asn Leu Gly Asp Pro Ile Tyr Gln Leu Ile Asn Lys
1655            1660            1665

Thr Lys Val Gln Val Lys Ile Asn Lys Ser Asp Leu Phe Lys Asp
1670            1675            1680

Met Lys Leu Val Glu Ile Lys Lys Ser Asp Ser Ser Tyr Gln Glu
1685            1690            1695

Ile Tyr Glu Leu Glu Glu Ala Asn Ile Lys Ser Ala Gln Pro Lys
1700            1705            1710

Ile Ile Val Glu Lys Gly Asp Lys Trp Thr Ala Ile Lys Arg Pro
1715            1720            1725

25

Ser Met Ile Phe Arg Tyr Asp Ala Glu Asn Asn Glu Pro Gln Ala
1730 1735 1740

Gly Gly Gly Ser Gly Gly Ser Glu Gln Lys Leu Ile Ser Glu Glu
1745 1750 1755

Asp Gly Gly Gly Gly Gly Gly Ser Gly Gly Gly Gly Ser Gly Gly
1760 1765 1770

Gly Gly Ser Gly Gly Gly Gly Gly Met Ser Ile Gln His Phe Arg
1775 1780 1785

Val Ala Leu Ile Pro Phe Phe Ala Ala Phe Cys Leu Pro Val Phe
1790 1795 1800

Ala His Pro Glu Thr Leu Val Lys Val Lys Asp Ala Glu Asp Gln
1805 1810 1815

Leu Gly Ala Arg Val Gly Tyr Ile Glu Leu Asp Leu Asn Ser Gly
1820 1825 1830

Lys Ile Leu Glu Ser Phe Arg Pro Glu Glu Arg Phe Pro Met Met
1835 1840 1845

Ser Thr Phe Lys Val Leu Leu Cys Gly Ala Val Leu Ser Arg Val
1850 1855 1860

Asp Ala Gly Gln Glu Gln Leu Gly Arg Arg Ile His Tyr Ser Gln
1865 1870 1875

Asn Asp Leu Val Glu Tyr Ser Pro Val Thr Glu Lys His Leu Thr
1880 1885 1890

Asp Gly Met Thr Val Arg Glu Leu Cys Ser Ala Ala Ile Thr Met
1895 1900 1905

Ser Asp Asn Thr Ala Ala Asn Leu Leu Leu Thr Thr Ile Gly Gly
1910 1915 1920

Pro Lys Glu Leu Thr Ala Phe Leu His Asn Met Gly Asp His Val
1925 1930 1935

Thr Arg Leu Asp Arg Trp Glu Pro Glu Leu Asn Glu Ala Ile Pro
1940 1945 1950

Asn Asp Glu Arg Asp Thr Thr Met Pro Ala Ala Met Ala Thr Thr
1955 1960 1965

Leu Arg Lys Leu Leu Thr Gly Glu Leu Leu Thr Leu Ala Ser Arg
1970 1975 1980

```
      Gln Gln  Leu Ile Asp Trp Met  Glu Ala Asp Lys Val  Ala Gly Pro
          1985                 1990                1995


      Leu Leu  Arg Ser Ala Leu Pro  Ala Gly Trp Phe Ile  Ala Asp Lys
          2000                 2005                2010


      Ser Gly  Ala Gly Glu Arg Gly  Ser Arg Gly Ile Ile  Ala Ala Leu
          2015                 2020                2025


      Gly Pro  Asp Gly Lys Pro Ser  Arg Ile Val Val Ile  Tyr Thr Thr
          2030                 2035                2040


      Gly Ser  Gln Ala Thr Met Asp  Glu Arg Asn Arg Gln  Ile Ala Glu
          2045                 2050                2055


      Ile Gly  Ala Ser Leu Ile Lys  His Trp
          2060                 2065
```

<210> 5
<211> 2067
<212> PRT
<213> artificial

<220>
<223> TEM1- GGSEQKLISEEDGG-BLIP (TEM1-c myc-BLIP)

<400> 5

```
Met Ser Ile Gln His Phe Arg Val Ala Leu Ile Pro Phe Phe Ala Ala
1               5                   10                  15

Phe Cys Leu Pro Val Phe Ala His Pro Glu Thr Leu Val Lys Val Lys
            20                  25                  30

Asp Ala Glu Asp Gln Leu Gly Ala Arg Val Gly Tyr Ile Glu Leu Asp
        35                  40                  45

Leu Asn Ser Gly Lys Ile Leu Glu Ser Phe Arg Pro Glu Glu Arg Phe
    50                  55                  60

Pro Met Met Ser Thr Phe Lys Val Leu Leu Cys Gly Ala Val Leu Ser
65                  70                  75                  80

Arg Val Asp Ala Gly Gln Glu Gln Leu Gly Arg Arg Ile His Tyr Ser
            85                  90                  95

Gln Asn Asp Leu Val Glu Tyr Ser Pro Val Thr Glu Lys His Leu Thr
            100                 105                 110

Asp Gly Met Thr Val Arg Glu Leu Cys Ser Ala Ala Ile Thr Met Ser
        115                 120                 125

Asp Asn Thr Ala Ala Asn Leu Leu Leu Thr Thr Ile Gly Gly Pro Lys
```

|  |  |  | 130 |  |  |  | 135 |  |  |  | 140 |  |  |  |
|---|---|---|---|---|---|---|---|---|---|---|---|---|---|---|

Glu Leu Thr Ala Phe Leu His Asn Met Gly Asp His Val Thr Arg Leu
145 150 155 160

Asp Arg Trp Glu Pro Glu Leu Asn Glu Ala Ile Pro Asn Asp Glu Arg
165 170 175

Asp Thr Thr Met Pro Ala Ala Met Ala Thr Thr Leu Arg Lys Leu Leu
180 185 190

Thr Gly Glu Leu Leu Thr Leu Ala Ser Arg Gln Gln Leu Ile Asp Trp
195 200 205

Met Glu Ala Asp Lys Val Ala Gly Pro Leu Leu Arg Ser Ala Leu Pro
210 215 220

Ala Gly Trp Phe Ile Ala Asp Lys Ser Gly Ala Gly Glu Arg Gly Ser
225 230 235 240

Arg Gly Ile Ile Ala Ala Leu Gly Pro Asp Gly Lys Pro Ser Arg Ile
245 250 255

Val Val Ile Tyr Thr Thr Gly Ser Gln Ala Thr Met Asp Glu Arg Asn
260 265 270

Arg Gln Ile Ala Glu Ile Gly Ala Ser Leu Ile Lys His Trp Gly Gly
275 280 285

Gly Ser Gly Gly Ser Glu Gln Lys Leu Ile Ser Glu Glu Asp Gly Gly
290 295 300

Gly Gly Gly Gly Ser Gly Gly Gly Gly Ser Gly Gly Gly Gly Ser Gly
305 310 315 320

Gly Gly Gly Gly Met Lys Gln Asn Lys Leu Leu Gln Arg Gly Ala Tyr
325 330 335

Phe Asn Asp Lys Asn Ile Leu Ile Asp Asp Phe Asp Lys Arg Tyr Asn
340 345 350

Asp Tyr Asp Phe Val Glu Phe Phe Thr Gly Ile Ser Asn Ser Thr Phe
355 360 365

Gly Leu Lys Ser Asp Gly Asn Leu Tyr Ala Cys Gly Asp Asn Thr Gly
370 375 380

Phe Gln Leu Gly Leu Gly Lys Asp Ser Ser Glu Arg Arg Met Phe Ser
385 390 395 400

Lys Val Lys Ile Asp Asn Val Lys Tyr Val Ser Cys Gly Ser Lys His

|  |  |  | 405 |  |  |  |  |  | 410 |  |  |  |  |  | 415 |

Ser Val Ala Val Thr Lys Asp Gly Phe Ala Tyr Gly Ala Gly Thr Ser
 420 425 430

Asn Val Gly Gln Leu Gly Val Ile Glu Ser Thr Val Tyr Tyr Glu Phe
 435 440 445

Thr Lys Leu Pro Ile Asp Asp Val Lys Thr Val Ala Cys Gly Tyr Asp
 450 455 460

Phe Thr Phe Val Leu Lys Asn Asp Gly Thr Leu Tyr Ser Ala Gly Leu
465 470 475 480

Asn Ser Ser Gly Gln Leu Gly Leu Gly Asp Thr Asn Asn Arg Val Thr
 485 490 495

Phe Thr Lys Val Asn Ile Asp Ser Val Lys Asp Val Val Thr Tyr Asn
 500 505 510

Gln Ser Val Phe Ile Ile Lys Met Asp Gly Thr Ala His Ala Cys Gly
 515 520 525

Leu Asn Ser Asn Gly Gln Leu Gly Ile Asn Ser Thr Leu Asn Lys Ser
 530 535 540

Val Phe Asn Lys Ile Glu Gly Met Asp Asn Val Lys Gln Ile Ala Cys
545 550 555 560

Gly Ser Ser His Thr Ile Leu Ile Lys Asn Asp Gly Thr Met Tyr Thr
 565 570 575

Thr Gly Ser Asn Gly Tyr Gly Gln Leu Gly Thr Gly Asn Asn Asn Asn
 580 585 590

Ser Ile Val Phe Thr Leu Ser Ser Ile Asn Asn Val Lys Tyr Ala Ser
 595 600 605

Cys Gly Asn Asn His Thr Met Ile Leu Lys Tyr Asp Asn Thr Leu Phe
 610 615 620

Ser Thr Gly Gln Asn Asn Tyr Gly Gln Leu Ala Asn Ala Asn Lys Asp
625 630 635 640

Val Ala Ser Arg Asn Thr Phe Val Lys Val Asn Val Glu Asn Ile Lys
 645 650 655

Asp Ile Lys Cys Gly Ser Gln Phe Asn Phe Leu Ile Asn Gly Ser Lys
 660 665 670

Glu Ile Phe Val Ser Gly Cys Asn Leu Ala Gly Gln Leu Gly Ser Phe

```
                    675                     680                     685

        Phe His Thr Thr Phe Leu Tyr Glu Phe Ser Lys Val Gln Ser Ser Asn
            690                 695                 700

        Leu Asp Asn Tyr Ser Gly Leu Leu Val Asn Asp Asp Tyr Leu Tyr Val
        705                 710                 715                 720

        Thr Lys Asp Asn Ser Glu Phe Leu Asn Val Lys Leu Ser Asp Asn Phe
                            725                 730                 735

        Gln Asp Tyr Lys Lys Ile Glu Leu Thr Asp Asn Asn Met Phe Ile Val
                    740                 745                 750

        Met Asn Asp Gly Thr Leu Tyr Ala Cys Gly Leu Asn Asn Tyr Gly Gln
                    755                 760                 765

        Leu Gly Leu Gly Asp Thr Val Asn Arg Ser Val Met Thr Lys Val Asp
            770                 775                 780

        Ile Asp Asn Val Leu Asp Ile Lys Gly Asn Gly Asn Ser Thr Phe Val
        785                 790                 795                 800

        Leu Lys Asn Asn Gly Thr Leu Tyr Ser Cys Gly Tyr Asn Ser Ser Gly
                        805                 810                 815

        Ile Leu Gly Leu Lys Asp Asn Thr Asn Arg Asn Ile Phe Thr Lys Ile
                    820                 825                 830

        Glu Ile Glu Asn Ile Lys Glu Phe Cys Val Glu Ser Asn Tyr Ile Val
                835                 840                 845

        Ala Leu Asn His Ser Lys Glu Leu Tyr Gly Trp Gly Asn Gln Ser Tyr
            850                 855                 860

        Ile Val Tyr Gly Asp Asn Arg Asn Tyr Pro Tyr Lys Asp Thr Arg Val
        865                 870                 875                 880

        Ser Asn Val Glu Lys Ile Ala Thr Trp Ser Asp Thr Leu Tyr Ile Leu
                        885                 890                 895

        Asp Ser Thr Gly Ala Thr Lys Thr Ile Gly Tyr Ser Tyr Asn Gly Ser
                    900                 905                 910

        Gly Gly Tyr Pro Ala Pro Ser Ser Ser Ser Thr Tyr Arg Glu Gly Gly
                    915                 920                 925

        Tyr Ile Asn Lys Asn Thr Ser Tyr Arg Thr Leu Glu Phe Tyr Asn Thr
            930                 935                 940

        Ser Lys Thr Lys Leu Val Asn Leu Phe Ala Phe Tyr Asn Gly Cys Val
```

945 950 955 960

Phe Val Asp Glu Asn Gly Leu Ala Tyr Cys Ile Gly Glu Asn Asn Ile
965 970 975

Asn Phe Arg Gly Gly Ser Thr Thr Asn Glu Asn Asn Ser Leu Arg Phe
980 985 990

Ile Asn Asn Ser Gly Val Tyr Tyr Thr Asn Thr Asp Gly Thr Asp Tyr
995 1000 1005

Thr Cys Tyr Gln Trp Thr Tyr Lys Leu Ile Arg Cys Ser Ile Phe
1010 1015 1020

Asp Ser Pro Gln Asn Ile Ile Gly Asn Ser Lys Asn Ile Leu Tyr
1025 1030 1035

Leu Ser Lys Asn Asn Ser Thr Phe Lys Cys Thr Gly Asn Cys Ile
1040 1045 1050

Thr Tyr Gly Ile Asn Ser Gln Asn Trp Tyr Ser Tyr Phe Ser Asp
1055 1060 1065

Ser Ser Asn Gly Ala Ile Ala Leu Gly Asn Glu Phe Ile Leu Lys
1070 1075 1080

Asn Tyr Ser Gly Glu Cys Leu Leu Lys Gly Tyr Gly Lys Ala Thr
1085 1090 1095

Asn Gly Glu Phe Gly Asn Ser Thr Asn Ile Ser Ser Ile Ser Asn
1100 1105 1110

Tyr Asp Thr Gly Leu Lys Asp Ile Lys Asp Ile Ile Val Lys Asn
1115 1120 1125

Asn Thr Val Val Val Val Asp Lys Asn Asn Asn Ile Tyr Val Thr
1130 1135 1140

Gly Ala Asn Gln Phe Asn Lys Leu Gly Ile Gly Glu Tyr Asn Asn
1145 1150 1155

Gln Pro Ile Arg Lys Phe Thr Asn Ile Thr Glu Gln Ser Asn Ser
1160 1165 1170

Phe Ile Phe Met Asp Asp Ile Lys Glu Ile Thr Thr Ser Arg Asn
1175 1180 1185

Thr Met Phe Ile Val Lys Asn Asp Gly Thr Ala Tyr Ala Thr Gly
1190 1195 1200

Asn Asn Ser Ser Gly Gln Leu Gly Leu Gly Asp Thr Ile Asn Arg

```
                1205                      1210                      1215
```

Asn Lys Phe Thr Gln Ile Asn Leu Asp Asn Ile Lys Lys Ile Ser
    1220            1225            1230

Thr Ser Ile Asp Gly Asn Thr Thr Phe Ala Ile Arg Asn Asp Gly
    1235            1240            1245

Thr Leu Tyr Ser Thr Gly Leu Asn Thr Lys Gly Gln Leu Gly Leu
    1250            1255            1260

Gly Asp Ile Val Asn Arg Asn Thr Phe Thr Lys Val Asn Ile Gln
    1265            1270            1275

Asn Val Arg Asp Val Val Leu Gly Thr Thr His Ser His Ala Ile
    1280            1285            1290

Lys Asp Asp Asn Thr Leu Tyr Ser Cys Gly Glu Asn Thr His Gly
    1295            1300            1305

Gln Leu Gly Leu Gly Ser Glu Ser Asn His Pro Asp Val Leu Thr
    1310            1315            1320

Phe Thr Val Asn Asn Ile Thr Asn Val Arg Asp Val Tyr Cys Ser
    1325            1330            1335

Asp Thr Thr Thr Phe Ile Val Lys Asp Thr Asn Ile Ala Tyr Cys
    1340            1345            1350

Cys Gly Tyr Asn Asn Asn Ser Gln Leu Gly Met Gly Asn Thr Thr
    1355            1360            1365

Asp Gln Tyr Ser Phe Ile Lys Cys Met Glu Asn Val Lys Glu Val
    1370            1375            1380

Ile Pro Asn Glu Ile Asn Thr Tyr Ile Ile Thr Ile Tyr Asn Thr
    1385            1390            1395

Ala Tyr Ser Thr Gly Leu Asn Thr Asp Tyr Cys Leu Gly Leu Asn
    1400            1405            1410

Ser Asn Ser Asn Gln Ser Ser Phe Ser Glu Ile Pro Ile Ser Asn
    1415            1420            1425

Val Val Lys Val Ala Pro Asn Arg Asn Asn Ala Val Leu Leu Leu
    1430            1435            1440

Thr Ser Glu Gly Asp Val Tyr Thr Ala Gly Lys Cys Ser Asn Gly
    1445            1450            1455

Ser Gly Thr Gly Ser Glu Thr Pro Glu Lys Ile Lys Lys Ile Ala

33

|  | 1460 |  |  |  |  | 1465 |  |  |  |  | 1470 |  |  |  |
|---|---|---|---|---|---|---|---|---|---|---|---|---|---|---|

Ser Lys Ala Lys Asp Ile Gly Met Asn Tyr Arg Cys Gly His Tyr
    1475              1480              1485

Val Ser Asp Asn Gly Asp Leu Tyr Gly Thr Gly Phe Asn Asp Cys
    1490              1495              1500

Gly Gln Leu Gly Val Gly Asn Val Thr Lys Arg Asp Thr Phe Ile
    1505              1510              1515

Lys Thr Asn Thr Arg Val Lys Lys Ile Leu Pro Leu Glu Tyr Ala
    1520              1525              1530

Asn Ile Ala Ile Lys Asp Thr Asn Asp Ile Tyr Ile Cys Gly Leu
    1535              1540              1545

Asn Asn Tyr Gly Gln Leu Gly Val Gly Asn Arg Tyr Asp Ser Arg
    1550              1555              1560

Asn Asn Asp Asn Arg Ile Phe Asn Tyr Lys His Met Asn Phe Val
    1565              1570              1575

Met Gly Asp Leu Thr Ser Ile Lys Asn Arg His Asn Phe Ile Leu
    1580              1585              1590

Leu Asn Asn Lys Ile Val Ile Pro Thr Thr Lys Asp Ile Asp Tyr
    1595              1600              1605

Gly Leu Val Leu Gly Asn Leu Tyr Lys Gly Asp Leu Tyr Thr Glu
    1610              1615              1620

Leu Pro Tyr Glu Asp Ile Lys Glu Val Ser Ile Ser Lys Thr His
    1625              1630              1635

Ile Ile Ile Leu Leu Asn Asp Gly Thr Met Tyr Gly Cys Gly Thr
    1640              1645              1650

Asn Tyr His Gly Glu Leu Leu Gln Asp Leu Ser Ile Asn Gln Val
    1655              1660              1665

Asp Glu Phe Val Gln Ile Asn Val Ser Asp Val Lys His Val Ser
    1670              1675              1680

Cys Gly Asp Asn Phe Thr Tyr Phe Ile Lys Ser Asp Asp Ser Leu
    1685              1690              1695

Trp Ser Ile Gly Lys Asn Ser Glu Tyr Gln Leu Gly Ile Gly His
    1700              1705              1710

Asn Asn Pro Val Thr Glu Leu Gln Arg Ile Thr Thr Ile Ser Ser

|  |  | 1715 |  |  |  |  | 1720 |  |  |  |  | 1725 |  |  |  |
|---|---|---|---|---|---|---|---|---|---|---|---|---|---|---|---|

Cys Lys Glu Val His Cys Gly Lys Asn Tyr Thr Leu Val Val Thr
    1730                 1735                 1740

Thr Ser Asn Glu Leu Phe Val Gln Gly Tyr Asn Asp Lys Gly Ala
    1745                 1750                 1755

Leu Gly Leu Gly Ser Asp Ser Glu Asn Thr Ile Ile Lys Phe Phe
    1760                 1765                 1770

Thr Lys Ala Leu Thr Asp Ile Arg Glu Ile Lys Ser Tyr Gly Ser
    1775                 1780                 1785

Asp His Ile Leu Val Leu Lys Asn Asp Asn Ser Val Trp Val Thr
    1790                 1795                 1800

Gly Lys Asn Arg Asp Val Tyr Lys Ile Glu Gln Pro Val Glu Phe
    1805                 1810                 1815

Leu Lys Glu Phe Thr Ile Val Pro Ile Ser Glu Asp Val Asn Thr
    1820                 1825                 1830

Val Lys Asp Val Leu Ala Thr Asp Asn Thr Leu Tyr Ile Ile Ser
    1835                 1840                 1845

Glu Val Gly Thr Thr Asn Ala Ala Ile Glu Ile Thr Glu Lys Ser
    1850                 1855                 1860

Ile Ser Ser Ile Lys Ile Lys Ile Gln Asp Pro Asn Lys Asp Ile
    1865                 1870                 1875

Ser Arg Ile Glu Met Leu Ile Asn Gly Glu Ser Val Lys Ser Val
    1880                 1885                 1890

Ser Asp Leu Ile Thr Glu Lys Ile Ser Phe Glu Val Pro Pro Asp
    1895                 1900                 1905

Lys Ile Lys Ile Gly Glu Asn Lys Ile Leu Phe Arg Ala Tyr Cys
    1910                 1915                 1920

Lys Gly Asp Asp Leu Tyr Ala Ser Leu Phe Ile Phe Lys Glu Ser
    1925                 1930                 1935

Thr Gly Asn Ser Ile Ile Lys Asp Ser Tyr Val Met Ile Gly Asn
    1940                 1945                 1950

Arg Met Tyr Lys Val Val Asn Thr Thr Ser Asn Glu Gln Asp Ile
    1955                 1960                 1965

Thr Ile Thr Leu Asp Arg Gly Leu Glu Glu Asp Leu Asn Leu Gly

```
            1970                    1975                    1980
```

```
      Asp Pro  Ile Tyr Gln Leu Ile  Asn Lys Thr Lys Val  Gln Val Lys
          1985                1990                1995
```

```
      Ile Asn  Lys Ser Asp Leu Phe  Lys Asp Met Lys Leu  Val Glu Ile
          2000                2005                2010
```

```
      Lys Lys  Ser Asp Ser Ser Tyr  Gln Glu Ile Tyr Glu  Leu Glu Glu
          2015                2020                2025
```

```
      Ala Asn  Ile Lys Ser Ala Gln  Pro Lys Ile Ile Val  Glu Lys Gly
          2030                2035                2040
```

```
      Asp Lys  Trp Thr Ala Ile Lys  Arg Pro Ser Met Ile  Phe Arg Tyr
          2045                2050                2055
```

```
      Asp Ala  Glu Asn Asn Glu Pro  Gln Ala
          2060                2065
```

<210> 6
<211> 4
<212> PRT
<213> artificial

<220>
<223> Linker

<400> 6

```
                              Gly Gly Gly Ser
                              1
```

<210> 7
<211> 20
<212> PRT
<213> artificial

<220>
<223> Linker

<400> 7

```
      Gly Gly Gly Gly Ser Gly Gly Gly Gly Ser Gly Gly Gly Gly Ser Gly
      1                 5                 10                  15
```

```
      Gly Gly Gly Gly
                  20
```

<210> 8
<211> 6
<212> PRT
<213> Human immunodeficiency virus

<400> 8

Glu Lys Ile Arg Leu Arg
1               5

<210> 9
<211> 7
<212> PRT
<213> Homo sapiens

<400> 9

Ser Asp Leu Trp Lys Leu Leu
1               5

<210> 10
<211> 491
<212> PRT
<213> Homo sapiens

<400> 10

```
Met Cys Asn Thr Asn Met Ser Val Pro Thr Asp Gly Ala Val Thr Thr
1               5               10              15

Ser Gln Ile Pro Ala Ser Glu Gln Glu Thr Leu Val Arg Pro Lys Pro
            20              25              30

Leu Leu Leu Lys Leu Leu Lys Ser Val Gly Ala Gln Lys Asp Thr Tyr
        35              40              45

Thr Met Lys Glu Val Leu Phe Tyr Leu Gly Gln Tyr Ile Met Thr Lys
    50              55              60

Arg Leu Tyr Asp Glu Lys Gln Gln His Ile Val Tyr Cys Ser Asn Asp
65              70              75              80

Leu Leu Gly Asp Leu Phe Gly Val Pro Ser Phe Ser Val Lys Glu His
            85              90              95

Arg Lys Ile Tyr Thr Met Ile Tyr Arg Asn Leu Val Val Val Asn Gln
            100             105             110

Gln Glu Ser Ser Asp Ser Gly Thr Ser Val Ser Glu Asn Arg Cys His
        115             120             125

Leu Glu Gly Gly Ser Asp Gln Lys Asp Leu Val Gln Glu Leu Gln Glu
    130             135             140

Glu Lys Pro Ser Ser Ser His Leu Val Ser Arg Pro Ser Thr Ser Ser
145             150             155             160

Arg Arg Arg Ala Ile Ser Glu Thr Glu Glu Asn Ser Asp Glu Leu Ser
            165             170             175

Gly Glu Arg Gln Arg Lys Arg His Lys Ser Asp Ser Ile Ser Leu Ser
        180             185             190
```

```
Phe Asp Glu Ser Leu Ala Leu Cys Val Ile Arg Glu Ile Cys Cys Glu
        195                 200                 205
```

```
Arg Ser Ser Ser Ser Glu Ser Thr Gly Thr Pro Ser Asn Pro Asp Leu
    210             215             220
```

```
Asp Ala Gly Val Ser Glu His Ser Gly Asp Trp Leu Asp Gln Asp Ser
225             230             235                 240
```

```
Val Ser Asp Gln Phe Ser Val Glu Phe Glu Val Glu Ser Leu Asp Ser
            245             250                 255
```

```
Glu Asp Tyr Ser Leu Ser Glu Glu Gly Gln Glu Leu Ser Asp Glu Asp
            260             265                 270
```

```
Asp Glu Val Tyr Gln Val Thr Val Tyr Gln Ala Gly Glu Ser Asp Thr
            275             280             285
```

```
Asp Ser Phe Glu Glu Asp Pro Glu Ile Ser Leu Ala Asp Tyr Trp Lys
    290             295             300
```

```
Cys Thr Ser Cys Asn Glu Met Asn Pro Pro Leu Pro Ser His Cys Asn
305             310             315                 320
```

```
Arg Cys Trp Ala Leu Arg Glu Asn Trp Leu Pro Glu Asp Lys Gly Lys
            325             330             335
```

```
Asp Lys Gly Glu Ile Ser Glu Lys Ala Lys Leu Glu Asn Ser Thr Gln
            340             345             350
```

```
Ala Glu Glu Gly Phe Asp Val Pro Asp Cys Lys Lys Thr Ile Val Asn
    355             360             365
```

```
Asp Ser Arg Glu Ser Cys Val Glu Glu Asn Asp Asp Lys Ile Thr Gln
    370             375             380
```

```
Ala Ser Gln Ser Gln Glu Ser Glu Asp Tyr Ser Gln Pro Ser Thr Ser
385             390             395                 400
```

```
Ser Ser Ile Ile Tyr Ser Ser Gln Glu Asp Val Lys Glu Phe Glu Arg
            405             410             415
```

```
Glu Glu Thr Gln Asp Lys Glu Glu Ser Val Glu Ser Ser Leu Pro Leu
        420             425             430
```

```
Asn Ala Ile Glu Pro Cys Val Ile Cys Gln Gly Arg Pro Lys Asn Gly
        435             440             445
```

```
Cys Ile Val His Gly Lys Thr Gly His Leu Met Ala Cys Phe Thr Cys
    450             455             460
```

```
Ala Lys Lys Leu Lys Lys Arg Asn Lys Pro Cys Pro Val Cys Arg Gln
    465                 470                 475                 480

Pro Ile Gln Met Ile Val Leu Thr Tyr Phe Pro
                485                 490
```

<210> 11
<211> 2060
<212> PRT
<213> artificial

<220>
<223> Tem1-SDLWKLL-BLIP

<400> 11

```
Met Ser Ile Gln His Phe Arg Val Ala Leu Ile Pro Phe Phe Ala Ala
1             5                 10                15

Phe Cys Leu Pro Val Phe Ala His Pro Glu Thr Leu Val Lys Val Lys
            20              25                  30

Asp Ala Glu Asp Gln Leu Gly Ala Arg Val Gly Tyr Ile Glu Leu Asp
        35                  40              45

Leu Asn Ser Gly Lys Ile Leu Glu Ser Phe Arg Pro Glu Glu Arg Phe
    50                  55                  60

Pro Met Met Ser Thr Phe Lys Val Leu Leu Cys Gly Ala Val Leu Ser
65                  70              75                  80

Arg Val Asp Ala Gly Gln Glu Gln Leu Gly Arg Arg Ile His Tyr Ser
            85                  90                  95

Gln Asn Asp Leu Val Glu Tyr Ser Pro Val Thr Glu Lys His Leu Thr
        100             105             110

Asp Gly Met Thr Val Arg Glu Leu Cys Ser Ala Ala Ile Thr Met Ser
        115             120             125

Asp Asn Thr Ala Ala Asn Leu Leu Leu Thr Thr Ile Gly Gly Pro Lys
    130             135             140

Glu Leu Thr Ala Phe Leu His Asn Met Gly Asp His Val Thr Arg Leu
145             150             155             160

Asp Arg Trp Glu Pro Glu Leu Asn Glu Ala Ile Pro Asn Asp Glu Arg
            165             170             175

Asp Thr Thr Met Pro Ala Ala Met Ala Thr Thr Leu Arg Lys Leu Leu
        180             185             190

Thr Gly Glu Leu Leu Thr Leu Ala Ser Arg Gln Gln Leu Ile Asp Trp
    195             200             205
```

41

Met Glu Ala Asp Lys Val Ala Gly Pro Leu Leu Arg Ser Ala Leu Pro
    210             215             220

Ala Gly Trp Phe Ile Ala Asp Lys Ser Gly Ala Gly Glu Arg Gly Ser
225             230             235             240

Arg Gly Ile Ile Ala Ala Leu Gly Pro Asp Gly Lys Pro Ser Arg Ile
            245             250             255

Val Val Ile Tyr Thr Thr Gly Ser Gln Ala Thr Met Asp Glu Arg Asn
            260             265             270

Arg Gln Ile Ala Glu Ile Gly Ala Ser Leu Ile Lys His Trp Gly Gly
        275             280             285

Gly Ser Ser Asp Leu Trp Lys Leu Leu Gly Gly Gly Gly Ser Gly Gly
    290             295             300

Gly Gly Ser Gly Gly Gly Gly Ser Gly Gly Gly Gly Met Lys Gln
305             310             315             320

Asn Lys Leu Leu Gln Arg Gly Ala Tyr Phe Asn Asp Lys Asn Ile Leu
            325             330             335

Ile Asp Asp Phe Asp Lys Arg Tyr Asn Asp Tyr Asp Phe Val Glu Phe
            340             345             350

Phe Thr Gly Ile Ser Asn Ser Thr Phe Gly Leu Lys Ser Asp Gly Asn
        355             360             365

Leu Tyr Ala Cys Gly Asp Asn Thr Gly Phe Gln Leu Gly Leu Gly Lys
    370             375             380

Asp Ser Ser Glu Arg Arg Met Phe Ser Lys Val Lys Ile Asp Asn Val
385             390             395             400

Lys Tyr Val Ser Cys Gly Ser Lys His Ser Val Ala Val Thr Lys Asp
            405             410             415

Gly Phe Ala Tyr Gly Ala Gly Thr Ser Asn Val Gly Gln Leu Gly Val
        420             425             430

Ile Glu Ser Thr Val Tyr Tyr Glu Phe Thr Lys Leu Pro Ile Asp Asp
        435             440             445

Val Lys Thr Val Ala Cys Gly Tyr Asp Phe Thr Phe Val Leu Lys Asn
    450             455             460

Asp Gly Thr Leu Tyr Ser Ala Gly Leu Asn Ser Ser Gly Gln Leu Gly
465             470             475             480

```
Leu Gly Asp Thr Asn Asn Arg Val Thr Phe Thr Lys Val Asn Ile Asp
            485                 490             495

Ser Val Lys Asp Val Val Thr Tyr Asn Gln Ser Val Phe Ile Ile Lys
            500                 505             510

Met Asp Gly Thr Ala His Ala Cys Gly Leu Asn Ser Asn Gly Gln Leu
            515             520             525

Gly Ile Asn Ser Thr Leu Asn Lys Ser Val Phe Asn Lys Ile Glu Gly
        530             535             540

Met Asp Asn Val Lys Gln Ile Ala Cys Gly Ser Ser His Thr Ile Leu
545             550             555                 560

Ile Lys Asn Asp Gly Thr Met Tyr Thr Thr Gly Ser Asn Gly Tyr Gly
                565                 570                 575

Gln Leu Gly Thr Gly Asn Asn Asn Ser Ile Val Phe Thr Leu Ser
            580             585             590

Ser Ile Asn Asn Val Lys Tyr Ala Ser Cys Gly Asn Asn His Thr Met
        595                 600             605

Ile Leu Lys Tyr Asp Asn Thr Leu Phe Ser Thr Gly Gln Asn Asn Tyr
        610             615             620

Gly Gln Leu Ala Asn Ala Asn Lys Asp Val Ala Ser Arg Asn Thr Phe
625                 630                 635                 640

Val Lys Val Asn Val Glu Asn Ile Lys Asp Ile Lys Cys Gly Ser Gln
                645                 650                 655

Phe Asn Phe Leu Ile Asn Gly Ser Lys Glu Ile Phe Val Ser Gly Cys
            660             665                 670

Asn Leu Ala Gly Gln Leu Gly Ser Phe Phe His Thr Thr Phe Leu Tyr
            675             680             685

Glu Phe Ser Lys Val Gln Ser Ser Asn Leu Asp Asn Tyr Ser Gly Leu
        690             695             700

Leu Val Asn Asp Asp Tyr Leu Tyr Val Thr Lys Asp Asn Ser Glu Phe
705                 710                 715                 720

Leu Asn Val Lys Leu Ser Asp Asn Phe Gln Asp Tyr Lys Lys Ile Glu
                725                 730                 735

Leu Thr Asp Asn Asn Met Phe Ile Val Met Asn Asp Gly Thr Leu Tyr
            740                 745                 750
```

Ala Cys Gly Leu Asn Asn Tyr Gly Gln Leu Gly Leu Gly Asp Thr Val
755 760 765

Asn Arg Ser Val Met Thr Lys Val Asp Ile Asp Asn Val Leu Asp Ile
770 775 780

Lys Gly Asn Gly Asn Ser Thr Phe Val Leu Lys Asn Asn Gly Thr Leu
785 790 795 800

Tyr Ser Cys Gly Tyr Asn Ser Ser Gly Ile Leu Gly Leu Lys Asp Asn
805 810 815

Thr Asn Arg Asn Ile Phe Thr Lys Ile Glu Ile Glu Asn Ile Lys Glu
820 825 830

Phe Cys Val Glu Ser Asn Tyr Ile Val Ala Leu Asn His Ser Lys Glu
835 840 845

Leu Tyr Gly Trp Gly Asn Gln Ser Tyr Ile Val Tyr Gly Asp Asn Arg
850 855 860

Asn Tyr Pro Tyr Lys Asp Thr Arg Val Ser Asn Val Glu Lys Ile Ala
865 870 875 880

Thr Trp Ser Asp Thr Leu Tyr Ile Leu Asp Ser Thr Gly Ala Thr Lys
885 890 895

Thr Ile Gly Tyr Ser Tyr Asn Gly Ser Gly Gly Tyr Pro Ala Pro Ser
900 905 910

Ser Ser Ser Thr Tyr Arg Glu Gly Gly Tyr Ile Asn Lys Asn Thr Ser
915 920 925

Tyr Arg Thr Leu Glu Phe Tyr Asn Thr Ser Lys Thr Lys Leu Val Asn
930 935 940

Leu Phe Ala Phe Tyr Asn Gly Cys Val Phe Val Asp Glu Asn Gly Leu
945 950 955 960

Ala Tyr Cys Ile Gly Glu Asn Asn Ile Asn Phe Arg Gly Gly Ser Thr
965 970 975

Thr Asn Glu Asn Asn Ser Leu Arg Phe Ile Asn Asn Ser Gly Val Tyr
980 985 990

Tyr Thr Asn Thr Asp Gly Thr Asp Tyr Thr Cys Tyr Gln Trp Thr Tyr
995 1000 1005

Lys Leu Ile Arg Cys Ser Ile Phe Asp Ser Pro Gln Asn Ile Ile
1010 1015 1020

```
Gly Asn Ser Lys Asn Ile Leu Tyr Leu Ser Lys Asn Asn Ser Thr
    1025                1030            1035

Phe Lys Cys Thr Gly Asn Cys Ile Thr Tyr Gly Ile Asn Ser Gln
    1040                1045            1050

Asn Trp Tyr Ser Tyr Phe Ser Asp Ser Ser Asn Gly Ala Ile Ala
    1055                1060            1065

Leu Gly Asn Glu Phe Ile Leu Lys Asn Tyr Ser Gly Glu Cys Leu
    1070                1075            1080

Leu Lys Gly Tyr Gly Lys Ala Thr Asn Gly Glu Phe Gly Asn Ser
    1085                1090            1095

Thr Asn Ile Ser Ser Ile Ser Asn Tyr Asp Thr Gly Leu Lys Asp
    1100                1105            1110

Ile Lys Asp Ile Ile Val Lys Asn Asn Thr Val Val Val Val Asp
    1115                1120            1125

Lys Asn Asn Asn Ile Tyr Val Thr Gly Ala Asn Gln Phe Asn Lys
    1130                1135            1140

Leu Gly Ile Gly Glu Tyr Asn Asn Gln Pro Ile Arg Lys Phe Thr
    1145                1150            1155

Asn Ile Thr Glu Gln Ser Asn Ser Phe Ile Phe Met Asp Asp Ile
    1160                1165            1170

Lys Glu Ile Thr Thr Ser Arg Asn Thr Met Phe Ile Val Lys Asn
    1175                1180            1185

Asp Gly Thr Ala Tyr Ala Thr Gly Asn Asn Ser Ser Gly Gln Leu
    1190                1195            1200

Gly Leu Gly Asp Thr Ile Asn Arg Asn Lys Phe Thr Gln Ile Asn
    1205                1210            1215

Leu Asp Asn Ile Lys Lys Ile Ser Thr Ser Ile Asp Gly Asn Thr
    1220                1225            1230

Thr Phe Ala Ile Arg Asn Asp Gly Thr Leu Tyr Ser Thr Gly Leu
    1235                1240            1245

Asn Thr Lys Gly Gln Leu Gly Leu Gly Asp Ile Val Asn Arg Asn
    1250                1255            1260

Thr Phe Thr Lys Val Asn Ile Gln Asn Val Arg Asp Val Val Leu
    1265                1270            1275
```

```
Gly Thr  Thr His Ser His Ala  Ile Lys Asp Asp Asn  Thr Leu Tyr
    1280             1285             1290

Ser Cys  Gly Glu Asn Thr His  Gly Gln Leu Gly Leu  Gly Ser Glu
    1295             1300             1305

Ser Asn  His Pro Asp Val Leu  Thr Phe Thr Val Asn  Asn Ile Thr
    1310             1315             1320

Asn Val  Arg Asp Val Tyr Cys  Ser Asp Thr Thr Thr  Phe Ile Val
    1325             1330             1335

Lys Asp  Thr Asn Ile Ala Tyr  Cys Cys Gly Tyr Asn  Asn Asn Ser
    1340             1345             1350

Gln Leu  Gly Met Gly Asn Thr  Thr Asp Gln Tyr Ser  Phe Ile Lys
    1355             1360             1365

Cys Met  Glu Asn Val Lys Glu  Val Ile Pro Asn Glu  Ile Asn Thr
    1370             1375             1380

Tyr Ile  Ile Thr Ile Tyr Asn  Thr Ala Tyr Ser Thr  Gly Leu Asn
    1385             1390             1395

Thr Asp  Tyr Cys Leu Gly Leu  Asn Ser Asn Ser Asn  Gln Ser Ser
    1400             1405             1410

Phe Ser  Glu Ile Pro Ile Ser  Asn Val Val Lys Val  Ala Pro Asn
    1415             1420             1425

Arg Asn  Asn Ala Val Leu Leu  Leu Thr Ser Glu Gly  Asp Val Tyr
    1430             1435             1440

Thr Ala  Gly Lys Cys Ser Asn  Gly Ser Gly Thr Gly  Ser Glu Thr
    1445             1450             1455

Pro Glu  Lys Ile Lys Lys Ile  Ala Ser Lys Ala Lys  Asp Ile Gly
    1460             1465             1470

Met Asn  Tyr Arg Cys Gly His  Tyr Val Ser Asp Asn  Gly Asp Leu
    1475             1480             1485

Tyr Gly  Thr Gly Phe Asn Asp  Cys Gly Gln Leu Gly  Val Gly Asn
    1490             1495             1500

Val Thr  Lys Arg Asp Thr Phe  Ile Lys Thr Asn Thr  Arg Val Lys
    1505             1510             1515

Lys Ile  Leu Pro Leu Glu Tyr  Ala Asn Ile Ala Ile  Lys Asp Thr
    1520             1525             1530
```

```
Asn Asp  Ile Tyr Ile Cys Gly  Leu Asn Asn Tyr Gly  Gln Leu Gly
    1535                1540               1545

Val Gly  Asn Arg Tyr Asp Ser  Arg Asn Asn Asp Asn  Arg Ile Phe
    1550                1555               1560

Asn Tyr  Lys His Met Asn Phe  Val Met Gly Asp Leu  Thr Ser Ile
    1565                1570               1575

Lys Asn  Arg His Asn Phe Ile  Leu Leu Asn Asn Lys  Ile Val Ile
    1580                1585               1590

Pro Thr  Thr Lys Asp Ile Asp  Tyr Gly Leu Val Leu  Gly Asn Leu
    1595                1600               1605

Tyr Lys  Gly Asp Leu Tyr Thr  Glu Leu Pro Tyr Glu  Asp Ile Lys
    1610                1615               1620

Glu Val  Ser Ile Ser Lys Thr  His Ile Ile Ile Leu  Leu Asn Asp
    1625                1630               1635

Gly Thr  Met Tyr Gly Cys Gly  Thr Asn Tyr His Gly  Glu Leu Leu
    1640                1645               1650

Gln Asp  Leu Ser Ile Asn Gln  Val Asp Glu Phe Val  Gln Ile Asn
    1655                1660               1665

Val Ser  Asp Val Lys His Val  Ser Cys Gly Asp Asn  Phe Thr Tyr
    1670                1675               1680

Phe Ile  Lys Ser Asp Asp Ser  Leu Trp Ser Ile Gly  Lys Asn Ser
    1685                1690               1695

Glu Tyr  Gln Leu Gly Ile Gly  His Asn Asn Pro Val  Thr Glu Leu
    1700                1705               1710

Gln Arg  Ile Thr Thr Ile Ser  Ser Cys Lys Glu Val  His Cys Gly
    1715                1720               1725

Lys Asn  Tyr Thr Leu Val Val  Thr Thr Ser Asn Glu  Leu Phe Val
    1730                1735               1740

Gln Gly  Tyr Asn Asp Lys Gly  Ala Leu Gly Leu Gly  Ser Asp Ser
    1745                1750               1755

Glu Asn  Thr Ile Ile Lys Phe  Phe Thr Lys Ala Leu  Thr Asp Ile
    1760                1765               1770

Arg Glu  Ile Lys Ser Tyr Gly  Ser Asp His Ile Leu  Val Leu Lys
    1775                1780               1785
```

47

```
Asn Asp  Asn Ser Val Trp Val  Thr Gly Lys Asn Arg  Asp Val Tyr
    1790                 1795              1800

Lys Ile  Glu Gln Pro Val Glu  Phe Leu Lys Glu Phe  Thr Ile Val
    1805                 1810              1815

Pro Ile  Ser Glu Asp Val Asn  Thr Val Lys Asp Val  Leu Ala Thr
    1820                 1825              1830

Asp Asn  Thr Leu Tyr Ile Ile  Ser Glu Val Gly Thr  Thr Asn Ala
    1835                 1840              1845

Ala Ile  Glu Ile Thr Glu Lys  Ser Ile Ser Ser Ile  Lys Ile Lys
    1850                 1855              1860

Ile Gln  Asp Pro Asn Lys Asp  Ile Ser Arg Ile Glu  Met Leu Ile
    1865                 1870              1875

Asn Gly  Glu Ser Val Lys Ser  Val Ser Asp Leu Ile  Thr Glu Lys
    1880                 1885              1890

Ile Ser  Phe Glu Val Pro Pro  Asp Lys Ile Lys Ile  Gly Glu Asn
    1895                 1900              1905

Lys Ile  Leu Phe Arg Ala Tyr  Cys Lys Gly Asp Asp  Leu Tyr Ala
    1910                 1915              1920

Ser Leu  Phe Ile Phe Lys Glu  Ser Thr Gly Asn Ser  Ile Ile Lys
    1925                 1930              1935

Asp Ser  Tyr Val Met Ile Gly  Asn Arg Met Tyr Lys  Val Val Asn
    1940                 1945              1950

Thr Thr  Ser Asn Glu Gln Asp  Ile Thr Ile Thr Leu  Asp Arg Gly
    1955                 1960              1965

Leu Glu  Glu Asp Leu Asn Leu  Gly Asp Pro Ile Tyr  Gln Leu Ile
    1970                 1975              1980

Asn Lys  Thr Lys Val Gln Val  Lys Ile Asn Lys Ser  Asp Leu Phe
    1985                 1990              1995

Lys Asp  Met Lys Leu Val Glu  Ile Lys Lys Ser Asp  Ser Ser Tyr
    2000                 2005              2010

Gln Glu  Ile Tyr Glu Leu Glu  Glu Ala Asn Ile Lys  Ser Ala Gln
    2015                 2020              2025

Pro Lys  Ile Ile Val Glu Lys  Gly Asp Lys Trp Thr  Ala Ile Lys
    2030                 2035              2040
```

48

Arg Pro  Ser Met Ile Phe Arg  Tyr Asp Ala Glu Asn  Asn Glu Pro
    2045                  2050              2055

Gln Ala
    2060

<210> 12
<211> 7
<212> PRT
<213> Homo sapiens

<400> 12

Ser Gly Leu Trp Lys Leu Leu
1                5

<210> 13
<211> 7
<212> PRT
<213> Homo sapiens

<400> 13

Ala Gly Leu Trp Lys Leu Leu
1                5

<210> 14
<211> 2060
<212> PRT
<213> artificial

<220>
<223> TEM1-SGLWKLL-BLIP

<400> 14

```
Met Ser Ile Gln His Phe Arg Val Ala Leu Ile Pro Phe Phe Ala Ala
1               5               10              15

Phe Cys Leu Pro Val Phe Ala His Pro Glu Thr Leu Val Lys Val Lys
            20              25              30

Asp Ala Glu Asp Gln Leu Gly Ala Arg Val Gly Tyr Ile Glu Leu Asp
        35              40              45

Leu Asn Ser Gly Lys Ile Leu Glu Ser Phe Arg Pro Glu Glu Arg Phe
        50              55              60

Pro Met Met Ser Thr Phe Lys Val Leu Leu Cys Gly Ala Val Leu Ser
65              70              75              80

Arg Val Asp Ala Gly Gln Glu Gln Leu Gly Arg Arg Ile His Tyr Ser
            85              90              95

Gln Asn Asp Leu Val Glu Tyr Ser Pro Val Thr Glu Lys His Leu Thr
            100             105             110
```

```
Asp Gly Met Thr Val Arg Glu Leu Cys Ser Ala Ala Ile Thr Met Ser
        115             120             125

Asp Asn Thr Ala Ala Asn Leu Leu Leu Thr Thr Ile Gly Gly Pro Lys
        130             135             140

Glu Leu Thr Ala Phe Leu His Asn Met Gly Asp His Val Thr Arg Leu
145             150             155             160

Asp Arg Trp Glu Pro Glu Leu Asn Glu Ala Ile Pro Asn Asp Glu Arg
            165             170             175

Asp Thr Thr Met Pro Ala Ala Met Ala Thr Thr Leu Arg Lys Leu Leu
            180             185             190

Thr Gly Glu Leu Leu Thr Leu Ala Ser Arg Gln Gln Leu Ile Asp Trp
        195             200             205

Met Glu Ala Asp Lys Val Ala Gly Pro Leu Leu Arg Ser Ala Leu Pro
    210             215             220

Ala Gly Trp Phe Ile Ala Asp Lys Ser Gly Ala Gly Glu Arg Gly Ser
225             230             235             240

Arg Gly Ile Ile Ala Ala Leu Gly Pro Asp Gly Lys Pro Ser Arg Ile
            245             250             255

Val Val Ile Tyr Thr Thr Gly Ser Gln Ala Thr Met Asp Glu Arg Asn
            260             265             270

Arg Gln Ile Ala Glu Ile Gly Ala Ser Leu Ile Lys His Trp Gly Gly
        275             280             285

Gly Ser Ser Gly Leu Trp Lys Leu Leu Gly Gly Gly Gly Ser Gly Gly
    290             295             300

Gly Gly Ser Gly Gly Gly Gly Ser Gly Gly Gly Gly Met Lys Gln
305             310             315             320

Asn Lys Leu Leu Gln Arg Gly Ala Tyr Phe Asn Asp Lys Asn Ile Leu
            325             330             335

Ile Asp Asp Phe Asp Lys Arg Tyr Asn Asp Tyr Asp Phe Val Glu Phe
            340             345             350

Phe Thr Gly Ile Ser Asn Ser Thr Phe Gly Leu Lys Ser Asp Gly Asn
        355             360             365

Leu Tyr Ala Cys Gly Asp Asn Thr Gly Phe Gln Leu Gly Leu Gly Lys
    370             375             380
```

Asp Ser Ser Glu Arg Arg Met Phe Ser Lys Val Lys Ile Asp Asn Val
385                 390                 395                 400

Lys Tyr Val Ser Cys Gly Ser Lys His Ser Val Ala Val Thr Lys Asp
            405                 410                 415

Gly Phe Ala Tyr Gly Ala Gly Thr Ser Asn Val Gly Gln Leu Gly Val
            420                 425                 430

Ile Glu Ser Thr Val Tyr Tyr Glu Phe Thr Lys Leu Pro Ile Asp Asp
            435                 440                 445

Val Lys Thr Val Ala Cys Gly Tyr Asp Phe Thr Phe Val Leu Lys Asn
    450                 455                 460

Asp Gly Thr Leu Tyr Ser Ala Gly Leu Asn Ser Ser Gly Gln Leu Gly
465                 470                 475                 480

Leu Gly Asp Thr Asn Asn Arg Val Thr Phe Thr Lys Val Asn Ile Asp
                485                 490                 495

Ser Val Lys Asp Val Val Thr Tyr Asn Gln Ser Val Phe Ile Ile Lys
            500                 505                 510

Met Asp Gly Thr Ala His Ala Cys Gly Leu Asn Ser Asn Gly Gln Leu
            515                 520                 525

Gly Ile Asn Ser Thr Leu Asn Lys Ser Val Phe Asn Lys Ile Glu Gly
    530                 535                 540

Met Asp Asn Val Lys Gln Ile Ala Cys Gly Ser Ser His Thr Ile Leu
545                 550                 555                 560

Ile Lys Asn Asp Gly Thr Met Tyr Thr Thr Gly Ser Asn Gly Tyr Gly
            565                 570                 575

Gln Leu Gly Thr Gly Asn Asn Asn Asn Ser Ile Val Phe Thr Leu Ser
            580                 585                 590

Ser Ile Asn Asn Val Lys Tyr Ala Ser Cys Gly Asn Asn His Thr Met
            595                 600                 605

Ile Leu Lys Tyr Asp Asn Thr Leu Phe Ser Thr Gly Gln Asn Asn Tyr
    610                 615                 620

Gly Gln Leu Ala Asn Ala Asn Lys Asp Val Ala Ser Arg Asn Thr Phe
625                 630                 635                 640

Val Lys Val Asn Val Glu Asn Ile Lys Asp Ile Lys Cys Gly Ser Gln
            645                 650                 655

52

```
Phe Asn Phe Leu Ile Asn Gly Ser Lys Glu Ile Phe Val Ser Gly Cys
            660                 665                 670

Asn Leu Ala Gly Gln Leu Gly Ser Phe Phe His Thr Thr Phe Leu Tyr
            675                 680                 685

Glu Phe Ser Lys Val Gln Ser Ser Asn Leu Asp Asn Tyr Ser Gly Leu
            690                 695                 700

Leu Val Asn Asp Asp Tyr Leu Tyr Val Thr Lys Asp Asn Ser Glu Phe
705                 710                 715                 720

Leu Asn Val Lys Leu Ser Asp Asn Phe Gln Asp Tyr Lys Lys Ile Glu
                725                 730                 735

Leu Thr Asp Asn Asn Met Phe Ile Val Met Asn Asp Gly Thr Leu Tyr
            740                 745                 750

Ala Cys Gly Leu Asn Asn Tyr Gly Gln Leu Gly Leu Gly Asp Thr Val
            755                 760                 765

Asn Arg Ser Val Met Thr Lys Val Asp Ile Asp Asn Val Leu Asp Ile
    770                 775                 780

Lys Gly Asn Gly Asn Ser Thr Phe Val Leu Lys Asn Asn Gly Thr Leu
785                 790                 795                 800

Tyr Ser Cys Gly Tyr Asn Ser Ser Gly Ile Leu Gly Leu Lys Asp Asn
                805                 810                 815

Thr Asn Arg Asn Ile Phe Thr Lys Ile Glu Ile Glu Asn Ile Lys Glu
            820                 825                 830

Phe Cys Val Glu Ser Asn Tyr Ile Val Ala Leu Asn His Ser Lys Glu
            835                 840                 845

Leu Tyr Gly Trp Gly Asn Gln Ser Tyr Ile Val Tyr Gly Asp Asn Arg
    850                 855                 860

Asn Tyr Pro Tyr Lys Asp Thr Arg Val Ser Asn Val Glu Lys Ile Ala
865                 870                 875                 880

Thr Trp Ser Asp Thr Leu Tyr Ile Leu Asp Ser Thr Gly Ala Thr Lys
                885                 890                 895

Thr Ile Gly Tyr Ser Tyr Asn Gly Ser Gly Gly Tyr Pro Ala Pro Ser
                900                 905                 910

Ser Ser Ser Thr Tyr Arg Glu Gly Gly Tyr Ile Asn Lys Asn Thr Ser
    915                 920                 925
```

```
Tyr Arg Thr Leu Glu Phe Tyr Asn Thr Ser Lys Thr Lys Leu Val Asn
    930                 935             940

Leu Phe Ala Phe Tyr Asn Gly Cys Val Phe Val Asp Glu Asn Gly Leu
945             950             955                 960

Ala Tyr Cys Ile Gly Glu Asn Asn Ile Asn Phe Arg Gly Gly Ser Thr
            965             970                 975

Thr Asn Glu Asn Asn Ser Leu Arg Phe Ile Asn Asn Ser Gly Val Tyr
        980             985             990

Tyr Thr Asn Thr Asp Gly Thr Asp Tyr Thr Cys Tyr Gln Trp Thr Tyr
        995             1000            1005

Lys Leu Ile Arg Cys Ser Ile Phe Asp Ser Pro Gln Asn Ile Ile
    1010            1015            1020

Gly Asn Ser Lys Asn Ile Leu Tyr Leu Ser Lys Asn Asn Ser Thr
    1025            1030            1035

Phe Lys Cys Thr Gly Asn Cys Ile Thr Tyr Gly Ile Asn Ser Gln
    1040            1045            1050

Asn Trp Tyr Ser Tyr Phe Ser Asp Ser Ser Asn Gly Ala Ile Ala
    1055            1060            1065

Leu Gly Asn Glu Phe Ile Leu Lys Asn Tyr Ser Gly Glu Cys Leu
    1070            1075            1080

Leu Lys Gly Tyr Gly Lys Ala Thr Asn Gly Glu Phe Gly Asn Ser
    1085            1090            1095

Thr Asn Ile Ser Ser Ile Ser Asn Tyr Asp Thr Gly Leu Lys Asp
    1100            1105            1110

Ile Lys Asp Ile Ile Val Lys Asn Asn Thr Val Val Val Val Asp
    1115            1120            1125

Lys Asn Asn Asn Ile Tyr Val Thr Gly Ala Asn Gln Phe Asn Lys
    1130            1135            1140

Leu Gly Ile Gly Glu Tyr Asn Asn Gln Pro Ile Arg Lys Phe Thr
    1145            1150            1155

Asn Ile Thr Glu Gln Ser Asn Ser Phe Ile Phe Met Asp Asp Ile
    1160            1165            1170

Lys Glu Ile Thr Thr Ser Arg Asn Thr Met Phe Ile Val Lys Asn
    1175            1180            1185
```

```
Asp Gly  Thr Ala Tyr Ala Thr  Gly Asn Asn Ser Ser  Gly Gln Leu
    1190                1195                1200

Gly Leu  Gly Asp Thr Ile Asn  Arg Asn Lys Phe Thr  Gln Ile Asn
    1205                1210                1215

Leu Asp  Asn Ile Lys Lys Ile  Ser Thr Ser Ile Asp  Gly Asn Thr
    1220                1225                1230

Thr Phe  Ala Ile Arg Asn Asp  Gly Thr Leu Tyr Ser  Thr Gly Leu
    1235                1240                1245

Asn Thr  Lys Gly Gln Leu Gly  Leu Gly Asp Ile Val  Asn Arg Asn
    1250                1255                1260

Thr Phe  Thr Lys Val Asn Ile  Gln Asn Val Arg Asp  Val Val Leu
    1265                1270                1275

Gly Thr  Thr His Ser His Ala  Ile Lys Asp Asp Asn  Thr Leu Tyr
    1280                1285                1290

Ser Cys  Gly Glu Asn Thr His  Gly Gln Leu Gly Leu  Gly Ser Glu
    1295                1300                1305

Ser Asn  His Pro Asp Val Leu  Thr Phe Thr Val Asn  Asn Ile Thr
    1310                1315                1320

Asn Val  Arg Asp Val Tyr Cys  Ser Asp Thr Thr Thr  Phe Ile Val
    1325                1330                1335

Lys Asp  Thr Asn Ile Ala Tyr  Cys Cys Gly Tyr Asn  Asn Asn Ser
    1340                1345                1350

Gln Leu  Gly Met Gly Asn Thr  Thr Asp Gln Tyr Ser  Phe Ile Lys
    1355                1360                1365

Cys Met  Glu Asn Val Lys Glu  Val Ile Pro Asn Glu  Ile Asn Thr
    1370                1375                1380

Tyr Ile  Ile Thr Ile Tyr Asn  Thr Ala Tyr Ser Thr  Gly Leu Asn
    1385                1390                1395

Thr Asp  Tyr Cys Leu Gly Leu  Asn Ser Asn Ser Asn  Gln Ser Ser
    1400                1405                1410

Phe Ser  Glu Ile Pro Ile Ser  Asn Val Val Lys Val  Ala Pro Asn
    1415                1420                1425

Arg Asn  Asn Ala Val Leu Leu  Leu Thr Ser Glu Gly  Asp Val Tyr
    1430                1435                1440
```

```
Thr Ala  Gly Lys Cys Ser Asn  Gly Ser Gly Thr Gly  Ser Glu Thr
    1445                 1450                 1455

Pro Glu  Lys Ile Lys Lys Ile  Ala Ser Lys Ala Lys  Asp Ile Gly
    1460                 1465                 1470

Met Asn  Tyr Arg Cys Gly His  Tyr Val Ser Asp Asn  Gly Asp Leu
    1475                 1480                 1485

Tyr Gly  Thr Gly Phe Asn Asp  Cys Gly Gln Leu Gly  Val Gly Asn
    1490                 1495                 1500

Val Thr  Lys Arg Asp Thr Phe  Ile Lys Thr Asn Thr  Arg Val Lys
    1505                 1510                 1515

Lys Ile  Leu Pro Leu Glu Tyr  Ala Asn Ile Ala Ile  Lys Asp Thr
    1520                 1525                 1530

Asn Asp  Ile Tyr Ile Cys Gly  Leu Asn Asn Tyr Gly  Gln Leu Gly
    1535                 1540                 1545

Val Gly  Asn Arg Tyr Asp Ser  Arg Asn Asn Asp Asn  Arg Ile Phe
    1550                 1555                 1560

Asn Tyr  Lys His Met Asn Phe  Val Met Gly Asp Leu  Thr Ser Ile
    1565                 1570                 1575

Lys Asn  Arg His Asn Phe Ile  Leu Leu Asn Asn Lys  Ile Val Ile
    1580                 1585                 1590

Pro Thr  Thr Lys Asp Ile Asp  Tyr Gly Leu Val Leu  Gly Asn Leu
    1595                 1600                 1605

Tyr Lys  Gly Asp Leu Tyr Thr  Glu Leu Pro Tyr Glu  Asp Ile Lys
    1610                 1615                 1620

Glu Val  Ser Ile Ser Lys Thr  His Ile Ile Ile Leu  Leu Asn Asp
    1625                 1630                 1635

Gly Thr  Met Tyr Gly Cys Gly  Thr Asn Tyr His Gly  Glu Leu Leu
    1640                 1645                 1650

Gln Asp  Leu Ser Ile Asn Gln  Val Asp Glu Phe Val  Gln Ile Asn
    1655                 1660                 1665

Val Ser  Asp Val Lys His Val  Ser Cys Gly Asp Asn  Phe Thr Tyr
    1670                 1675                 1680

Phe Ile  Lys Ser Asp Asp Ser  Leu Trp Ser Ile Gly  Lys Asn Ser
    1685                 1690                 1695
```

```
Glu Tyr Gln Leu Gly Ile Gly His Asn Asn Pro Val Thr Glu Leu
    1700            1705            1710

Gln Arg Ile Thr Thr Ile Ser Ser Cys Lys Glu Val His Cys Gly
    1715            1720            1725

Lys Asn Tyr Thr Leu Val Val Thr Thr Ser Asn Glu Leu Phe Val
    1730            1735            1740

Gln Gly Tyr Asn Asp Lys Gly Ala Leu Gly Leu Gly Ser Asp Ser
    1745            1750            1755

Glu Asn Thr Ile Ile Lys Phe Phe Thr Lys Ala Leu Thr Asp Ile
    1760            1765            1770

Arg Glu Ile Lys Ser Tyr Gly Ser Asp His Ile Leu Val Leu Lys
    1775            1780            1785

Asn Asp Asn Ser Val Trp Val Thr Gly Lys Asn Arg Asp Val Tyr
    1790            1795            1800

Lys Ile Glu Gln Pro Val Glu Phe Leu Lys Glu Phe Thr Ile Val
    1805            1810            1815

Pro Ile Ser Glu Asp Val Asn Thr Val Lys Asp Val Leu Ala Thr
    1820            1825            1830

Asp Asn Thr Leu Tyr Ile Ile Ser Glu Val Gly Thr Thr Asn Ala
    1835            1840            1845

Ala Ile Glu Ile Thr Glu Lys Ser Ile Ser Ser Ile Lys Ile Lys
    1850            1855            1860

Ile Gln Asp Pro Asn Lys Asp Ile Ser Arg Ile Glu Met Leu Ile
    1865            1870            1875

Asn Gly Glu Ser Val Lys Ser Val Ser Asp Leu Ile Thr Glu Lys
    1880            1885            1890

Ile Ser Phe Glu Val Pro Pro Asp Lys Ile Lys Ile Gly Glu Asn
    1895            1900            1905

Lys Ile Leu Phe Arg Ala Tyr Cys Lys Gly Asp Asp Leu Tyr Ala
    1910            1915            1920

Ser Leu Phe Ile Phe Lys Glu Ser Thr Gly Asn Ser Ile Ile Lys
    1925            1930            1935

Asp Ser Tyr Val Met Ile Gly Asn Arg Met Tyr Lys Val Val Asn
    1940            1945            1950
```

```
Thr Thr  Ser Asn Glu Gln Asp  Ile Thr Ile Thr Leu  Asp Arg Gly
    1955                 1960                 1965

Leu Glu  Glu Asp Leu Asn Leu  Gly Asp Pro Ile Tyr  Gln Leu Ile
    1970                 1975                 1980

Asn Lys  Thr Lys Val Gln Val  Lys Ile Asn Lys Ser  Asp Leu Phe
    1985                 1990                 1995

Lys Asp  Met Lys Leu Val Glu  Ile Lys Lys Ser Asp  Ser Ser Tyr
    2000                 2005                 2010

Gln Glu  Ile Tyr Glu Leu Glu  Glu Ala Asn Ile Lys  Ser Ala Gln
    2015                 2020                 2025

Pro Lys  Ile Ile Val Glu Lys  Gly Asp Lys Trp Thr  Ala Ile Lys
    2030                 2035                 2040

Arg Pro  Ser Met Ile Phe Arg  Tyr Asp Ala Glu Asn  Asn Glu Pro
    2045                 2050                 2055

Gln Ala
    2060
```

<210> 15
<211> 2060
<212> PRT
<213> artificial

<220>
<223> TEM1-AGLWKLL-BLIP

<400> 15

```
Met Ser Ile Gln His Phe Arg Val Ala Leu Ile Pro Phe Phe Ala Ala
1               5               10              15

Phe Cys Leu Pro Val Phe Ala His Pro Glu Thr Leu Val Lys Val Lys
            20              25              30

Asp Ala Glu Asp Gln Leu Gly Ala Arg Val Gly Tyr Ile Glu Leu Asp
        35              40              45

Leu Asn Ser Gly Lys Ile Leu Glu Ser Phe Arg Pro Glu Glu Arg Phe
    50              55              60

Pro Met Met Ser Thr Phe Lys Val Leu Leu Cys Gly Ala Val Leu Ser
65              70              75              80

Arg Val Asp Ala Gly Gln Glu Gln Leu Gly Arg Arg Ile His Tyr Ser
                85              90              95
```

```
            Gln Asn Asp Leu Val Glu Tyr Ser Pro Val Thr Glu Lys His Leu Thr
                        100                 105                 110

            Asp Gly Met Thr Val Arg Glu Leu Cys Ser Ala Ala Ile Thr Met Ser
                        115                 120                 125

            Asp Asn Thr Ala Ala Asn Leu Leu Leu Thr Thr Ile Gly Gly Pro Lys
                130                 135                 140

            Glu Leu Thr Ala Phe Leu His Asn Met Gly Asp His Val Thr Arg Leu
            145                 150                 155                 160

            Asp Arg Trp Glu Pro Glu Leu Asn Glu Ala Ile Pro Asn Asp Glu Arg
                            165                 170                 175

            Asp Thr Thr Met Pro Ala Ala Met Ala Thr Thr Leu Arg Lys Leu Leu
                        180                 185                 190

            Thr Gly Glu Leu Leu Thr Leu Ala Ser Arg Gln Gln Leu Ile Asp Trp
                    195                 200                 205

            Met Glu Ala Asp Lys Val Ala Gly Pro Leu Leu Arg Ser Ala Leu Pro
                210                 215                 220

            Ala Gly Trp Phe Ile Ala Asp Lys Ser Gly Ala Gly Glu Arg Gly Ser
            225                 230                 235                 240

            Arg Gly Ile Ile Ala Ala Leu Gly Pro Asp Gly Lys Pro Ser Arg Ile
                            245                 250                 255

            Val Val Ile Tyr Thr Thr Gly Ser Gln Ala Thr Met Asp Glu Arg Asn
                        260                 265                 270

            Arg Gln Ile Ala Glu Ile Gly Ala Ser Leu Ile Lys His Trp Gly Gly
                    275                 280                 285

            Gly Ser Ala Gly Leu Trp Lys Leu Leu Gly Gly Gly Gly Ser Gly Gly
                290                 295                 300

            Gly Gly Ser Gly Gly Gly Gly Ser Gly Gly Gly Gly Gly Met Lys Gln
            305                 310                 315                 320

            Asn Lys Leu Leu Gln Arg Gly Ala Tyr Phe Asn Asp Lys Asn Ile Leu
                            325                 330                 335

            Ile Asp Asp Phe Asp Lys Arg Tyr Asn Asp Tyr Asp Phe Val Glu Phe
                        340                 345                 350

            Phe Thr Gly Ile Ser Asn Ser Thr Phe Gly Leu Lys Ser Asp Gly Asn
                    355                 360                 365
```

```
Leu Tyr Ala Cys Gly Asp Asn Thr Gly Phe Gln Leu Gly Leu Gly Lys
    370               375                   380

Asp Ser Ser Glu Arg Arg Met Phe Ser Lys Val Lys Ile Asp Asn Val
385               390               395                   400

Lys Tyr Val Ser Cys Gly Ser Lys His Ser Val Ala Val Thr Lys Asp
                405               410                   415

Gly Phe Ala Tyr Gly Ala Gly Thr Ser Asn Val Gly Gln Leu Gly Val
            420               425               430

Ile Glu Ser Thr Val Tyr Tyr Glu Phe Thr Lys Leu Pro Ile Asp Asp
        435               440               445

Val Lys Thr Val Ala Cys Gly Tyr Asp Phe Thr Phe Val Leu Lys Asn
    450               455               460

Asp Gly Thr Leu Tyr Ser Ala Gly Leu Asn Ser Ser Gly Gln Leu Gly
465               470               475                   480

Leu Gly Asp Thr Asn Asn Arg Val Thr Phe Thr Lys Val Asn Ile Asp
            485               490               495

Ser Val Lys Asp Val Val Thr Tyr Asn Gln Ser Val Phe Ile Ile Lys
            500               505               510

Met Asp Gly Thr Ala His Ala Cys Gly Leu Asn Ser Asn Gly Gln Leu
            515               520               525

Gly Ile Asn Ser Thr Leu Asn Lys Ser Val Phe Asn Lys Ile Glu Gly
    530               535               540

Met Asp Asn Val Lys Gln Ile Ala Cys Gly Ser Ser His Thr Ile Leu
545               550               555                   560

Ile Lys Asn Asp Gly Thr Met Tyr Thr Thr Gly Ser Asn Gly Tyr Gly
            565               570               575

Gln Leu Gly Thr Gly Asn Asn Asn Ser Ile Val Phe Thr Leu Ser
            580               585               590

Ser Ile Asn Asn Val Lys Tyr Ala Ser Cys Gly Asn Asn His Thr Met
        595               600               605

Ile Leu Lys Tyr Asp Asn Thr Leu Phe Ser Thr Gly Gln Asn Asn Tyr
    610               615               620

Gly Gln Leu Ala Asn Ala Asn Lys Asp Val Ala Ser Arg Asn Thr Phe
625               630               635                   640
```

Val Lys Val Asn Val Glu Asn Ile Lys Asp Ile Lys Cys Gly Ser Gln
                    645                 650                 655

Phe Asn Phe Leu Ile Asn Gly Ser Lys Glu Ile Phe Val Ser Gly Cys
                660             665             670

Asn Leu Ala Gly Gln Leu Gly Ser Phe Phe His Thr Thr Phe Leu Tyr
            675             680             685

Glu Phe Ser Lys Val Gln Ser Ser Asn Leu Asp Asn Tyr Ser Gly Leu
    690             695             700

Leu Val Asn Asp Asp Tyr Leu Tyr Val Thr Lys Asp Asn Ser Glu Phe
705             710             715             720

Leu Asn Val Lys Leu Ser Asp Asn Phe Gln Asp Tyr Lys Lys Ile Glu
            725             730             735

Leu Thr Asp Asn Asn Met Phe Ile Val Met Asn Asp Gly Thr Leu Tyr
            740             745             750

Ala Cys Gly Leu Asn Asn Tyr Gly Gln Leu Gly Leu Gly Asp Thr Val
        755             760             765

Asn Arg Ser Val Met Thr Lys Val Asp Ile Asp Asn Val Leu Asp Ile
    770             775             780

Lys Gly Asn Gly Asn Ser Thr Phe Val Leu Lys Asn Asn Gly Thr Leu
785             790             795             800

Tyr Ser Cys Gly Tyr Asn Ser Ser Gly Ile Leu Gly Leu Lys Asp Asn
            805             810             815

Thr Asn Arg Asn Ile Phe Thr Lys Ile Glu Ile Glu Asn Ile Lys Glu
        820             825             830

Phe Cys Val Glu Ser Asn Tyr Ile Val Ala Leu Asn His Ser Lys Glu
        835             840             845

Leu Tyr Gly Trp Gly Asn Gln Ser Tyr Ile Val Tyr Gly Asp Asn Arg
    850             855             860

Asn Tyr Pro Tyr Lys Asp Thr Arg Val Ser Asn Val Glu Lys Ile Ala
865             870             875             880

Thr Trp Ser Asp Thr Leu Tyr Ile Leu Asp Ser Thr Gly Ala Thr Lys
            885             890             895

Thr Ile Gly Tyr Ser Tyr Asn Gly Ser Gly Gly Tyr Pro Ala Pro Ser
            900             905             910

Ser Ser Ser Thr Tyr Arg Glu Gly Gly Tyr Ile Asn Lys Asn Thr Ser
915              920                925

Tyr Arg Thr Leu Glu Phe Tyr Asn Thr Ser Lys Thr Lys Leu Val Asn
930              935              940

Leu Phe Ala Phe Tyr Asn Gly Cys Val Phe Val Asp Glu Asn Gly Leu
945              950              955              960

Ala Tyr Cys Ile Gly Glu Asn Asn Ile Asn Phe Arg Gly Gly Ser Thr
965              970              975

Thr Asn Glu Asn Asn Ser Leu Arg Phe Ile Asn Asn Ser Gly Val Tyr
980              985              990

Tyr Thr Asn Thr Asp Gly Thr Asp Tyr Thr Cys Tyr Gln Trp Thr Tyr
995              1000              1005

Lys Leu Ile Arg Cys Ser Ile Phe Asp Ser Pro Gln Asn Ile Ile
1010              1015              1020

Gly Asn Ser Lys Asn Ile Leu Tyr Leu Ser Lys Asn Asn Ser Thr
1025              1030              1035

Phe Lys Cys Thr Gly Asn Cys Ile Thr Tyr Gly Ile Asn Ser Gln
1040              1045              1050

Asn Trp Tyr Ser Tyr Phe Ser Asp Ser Ser Asn Gly Ala Ile Ala
1055              1060              1065

Leu Gly Asn Glu Phe Ile Leu Lys Asn Tyr Ser Gly Glu Cys Leu
1070              1075              1080

Leu Lys Gly Tyr Gly Lys Ala Thr Asn Gly Glu Phe Gly Asn Ser
1085              1090              1095

Thr Asn Ile Ser Ser Ile Ser Asn Tyr Asp Thr Gly Leu Lys Asp
1100              1105              1110

Ile Lys Asp Ile Ile Val Lys Asn Asn Thr Val Val Val Val Asp
1115              1120              1125

Lys Asn Asn Asn Ile Tyr Val Thr Gly Ala Asn Gln Phe Asn Lys
1130              1135              1140

Leu Gly Ile Gly Glu Tyr Asn Asn Gln Pro Ile Arg Lys Phe Thr
1145              1150              1155

Asn Ile Thr Glu Gln Ser Asn Ser Phe Ile Phe Met Asp Asp Ile
1160              1165              1170

```
Lys Glu Ile Thr Thr Ser Arg Asn Thr Met Phe Ile Val Lys Asn
        1175                1180                1185

Asp Gly Thr Ala Tyr Ala Thr Gly Asn Asn Ser Ser Gly Gln Leu
        1190                1195                1200

Gly Leu Gly Asp Thr Ile Asn Arg Asn Lys Phe Thr Gln Ile Asn
        1205                1210                1215

Leu Asp Asn Ile Lys Lys Ile Ser Thr Ser Ile Asp Gly Asn Thr
        1220                1225                1230

Thr Phe Ala Ile Arg Asn Asp Gly Thr Leu Tyr Ser Thr Gly Leu
        1235                1240                1245

Asn Thr Lys Gly Gln Leu Gly Leu Gly Asp Ile Val Asn Arg Asn
        1250                1255                1260

Thr Phe Thr Lys Val Asn Ile Gln Asn Val Arg Asp Val Val Leu
        1265                1270                1275

Gly Thr Thr His Ser His Ala Ile Lys Asp Asp Asn Thr Leu Tyr
        1280                1285                1290

Ser Cys Gly Glu Asn Thr His Gly Gln Leu Gly Leu Gly Ser Glu
        1295                1300                1305

Ser Asn His Pro Asp Val Leu Thr Phe Thr Val Asn Asn Ile Thr
        1310                1315                1320

Asn Val Arg Asp Val Tyr Cys Ser Asp Thr Thr Thr Phe Ile Val
        1325                1330                1335

Lys Asp Thr Asn Ile Ala Tyr Cys Cys Gly Tyr Asn Asn Asn Ser
        1340                1345                1350

Gln Leu Gly Met Gly Asn Thr Thr Asp Gln Tyr Ser Phe Ile Lys
        1355                1360                1365

Cys Met Glu Asn Val Lys Glu Val Ile Pro Asn Glu Ile Asn Thr
        1370                1375                1380

Tyr Ile Ile Thr Ile Tyr Asn Thr Ala Tyr Ser Thr Gly Leu Asn
        1385                1390                1395

Thr Asp Tyr Cys Leu Gly Leu Asn Ser Asn Ser Asn Gln Ser Ser
        1400                1405                1410

Phe Ser Glu Ile Pro Ile Ser Asn Val Val Lys Val Ala Pro Asn
        1415                1420                1425
```

Arg Asn Asn Ala Val Leu Leu Leu Thr Ser Glu Gly Asp Val Tyr
          1430                1435                1440

Thr Ala Gly Lys Cys Ser Asn Gly Ser Gly Thr Gly Ser Glu Thr
    1445                1450                1455

Pro Glu Lys Ile Lys Lys Ile Ala Ser Lys Ala Lys Asp Ile Gly
    1460                1465                1470

Met Asn Tyr Arg Cys Gly His Tyr Val Ser Asp Asn Gly Asp Leu
    1475                1480                1485

Tyr Gly Thr Gly Phe Asn Asp Cys Gly Gln Leu Gly Val Gly Asn
    1490                1495                1500

Val Thr Lys Arg Asp Thr Phe Ile Lys Thr Asn Thr Arg Val Lys
    1505                1510                1515

Lys Ile Leu Pro Leu Glu Tyr Ala Asn Ile Ala Ile Lys Asp Thr
    1520                1525                1530

Asn Asp Ile Tyr Ile Cys Gly Leu Asn Asn Tyr Gly Gln Leu Gly
    1535                1540                1545

Val Gly Asn Arg Tyr Asp Ser Arg Asn Asn Asp Asn Arg Ile Phe
    1550                1555                1560

Asn Tyr Lys His Met Asn Phe Val Met Gly Asp Leu Thr Ser Ile
    1565                1570                1575

Lys Asn Arg His Asn Phe Ile Leu Leu Asn Asn Lys Ile Val Ile
    1580                1585                1590

Pro Thr Thr Lys Asp Ile Asp Tyr Gly Leu Val Leu Gly Asn Leu
    1595                1600                1605

Tyr Lys Gly Asp Leu Tyr Thr Glu Leu Pro Tyr Glu Asp Ile Lys
    1610                1615                1620

Glu Val Ser Ile Ser Lys Thr His Ile Ile Ile Leu Leu Asn Asp
    1625                1630                1635

Gly Thr Met Tyr Gly Cys Gly Thr Asn Tyr His Gly Glu Leu Leu
    1640                1645                1650

Gln Asp Leu Ser Ile Asn Gln Val Asp Glu Phe Val Gln Ile Asn
    1655                1660                1665

Val Ser Asp Val Lys His Val Ser Cys Gly Asp Asn Phe Thr Tyr
    1670                1675                1680

```
Phe Ile  Lys Ser Asp Asp Ser  Leu Trp Ser Ile Gly  Lys Asn Ser
    1685                1690                1695

Glu Tyr  Gln Leu Gly Ile Gly  His Asn Asn Pro Val  Thr Glu Leu
    1700                1705                1710

Gln Arg  Ile Thr Thr Ile Ser  Ser Cys Lys Glu Val  His Cys Gly
    1715                1720                1725

Lys Asn  Tyr Thr Leu Val Val  Thr Thr Ser Asn Glu  Leu Phe Val
    1730                1735                1740

Gln Gly  Tyr Asn Asp Lys Gly  Ala Leu Gly Leu Gly  Ser Asp Ser
    1745                1750                1755

Glu Asn  Thr Ile Ile Lys Phe  Phe Thr Lys Ala Leu  Thr Asp Ile
    1760                1765                1770

Arg Glu  Ile Lys Ser Tyr Gly  Ser Asp His Ile Leu  Val Leu Lys
    1775                1780                1785

Asn Asp  Asn Ser Val Trp Val  Thr Gly Lys Asn Arg  Asp Val Tyr
    1790                1795                1800

Lys Ile  Glu Gln Pro Val Glu  Phe Leu Lys Glu Phe  Thr Ile Val
    1805                1810                1815

Pro Ile  Ser Glu Asp Val Asn  Thr Val Lys Asp Val  Leu Ala Thr
    1820                1825                1830

Asp Asn  Thr Leu Tyr Ile Ile  Ser Glu Val Gly Thr  Thr Asn Ala
    1835                1840                1845

Ala Ile  Glu Ile Thr Glu Lys  Ser Ile Ser Ser Ile  Lys Ile Lys
    1850                1855                1860

Ile Gln  Asp Pro Asn Lys Asp  Ile Ser Arg Ile Glu  Met Leu Ile
    1865                1870                1875

Asn Gly  Glu Ser Val Lys Ser  Val Ser Asp Leu Ile  Thr Glu Lys
    1880                1885                1890

Ile Ser  Phe Glu Val Pro Pro  Asp Lys Ile Lys Ile  Gly Glu Asn
    1895                1900                1905

Lys Ile  Leu Phe Arg Ala Tyr  Cys Lys Gly Asp Asp  Leu Tyr Ala
    1910                1915                1920

Ser Leu  Phe Ile Phe Lys Glu  Ser Thr Gly Asn Ser  Ile Ile Lys
    1925                1930                1935
```

```
Asp Ser  Tyr Val Met Ile Gly  Asn Arg Met Tyr Lys  Val Val Asn
    1940                  1945              1950

Thr Thr  Ser Asn Glu Gln Asp  Ile Thr Ile Thr Leu  Asp Arg Gly
    1955                  1960              1965

Leu Glu  Glu Asp Leu Asn Leu  Gly Asp Pro Ile Tyr  Gln Leu Ile
    1970                  1975              1980

Asn Lys  Thr Lys Val Gln Val  Lys Ile Asn Lys Ser  Asp Leu Phe
    1985                  1990              1995

Lys Asp  Met Lys Leu Val Glu  Ile Lys Lys Ser Asp  Ser Ser Tyr
    2000                  2005              2010

Gln Glu  Ile Tyr Glu Leu Glu  Glu Ala Asn Ile Lys  Ser Ala Gln
    2015                  2020              2025

Pro Lys  Ile Ile Val Glu Lys  Gly Asp Lys Trp Thr  Ala Ile Lys
    2030                  2035              2040

Arg Pro  Ser Met Ile Phe Arg  Tyr Asp Ala Glu Asn  Asn Glu Pro
    2045                  2050              2055

Gln Ala
    2060
```

## Claims

1. A fusion protein comprising a structure of

$$(A_n\text{-}B\text{-}A_x\text{-}C\text{-}A_m)_y$$

wherein

each A independently comprises a peptide or protein domain that binds an analyte of interest, wherein A comprises an antibody, antibody fragment, peptide aptamer, peptide antigen, or peptide antigen fragment, wherein the peptide antigen or peptide antigen fragment preferably comprises an antigenic determinant or epitope;
B is an enzyme or has enzymatic activity, and comprises an entity that can produce a detectable signal;
C is an inhibitor of B, and comprises an entity that, when A is not bound to the analyte of interest, binds to B and modulates the signal production by B;
n and m are each independently 0 or at least 1;
x is an integer of at least 1;
y is an integer of at least 1;
"-" represents a covalent bond or a linker comprising or consisting of one or more amino acids;
wherein B and C each independently comprises a peptide or protein domain,

wherein the length of the linker between the peptides or protein domains is selected such that it allows the interaction of B and C when A is not bound to the analyte of interest, and it prevents the interaction of B and C when A is bound

66

to the analyte of interest.

**2.** The fusion protein of claim 1, wherein B is a peptide or protein domain coupled to a substance that can produce the detectable signal.

**3.** The fusion protein of claim 1 or 2, wherein the binding of B and C is impaired when all A moieties of the fusion protein are bound to an analyte, optionally the same analyte.

**4.** The fusion protein of any one of claims 1 to 3, wherein the fusion protein consists of the structure

$$(B\text{-}A\text{-}C)_y.$$

**5.** The fusion protein of claim 1, wherein B comprises a chromophore or fluorophore and C comprises a substance that absorbs emission energy of the chromophore or fluorophore.

**6.** The fusion protein of any one of claims 1 to 5, wherein when more than one A is present in the fusion protein, each A is the same or different, wherein when the two or more A are different, they are arranged to bind to the same analyte.

**7.** The fusion protein of any one of claims 1 to 6, wherein

(1) B is beta lactamase TEM1 or homologs, fragments and variants thereof, wherein the homologs, fragments and variants at least partially retain enzymatic activity, wherein the beta lactamase TEM1 preferably comprises the amino acid sequence set forth in SEQ ID NO: 1 or homologs, fragments or variants thereof; and
(2) C is beta lactamase inhibitor protein (BLIP) or fragments, homologs and variants thereof that retain at least partially the binding activity for beta lactamase, wherein the BLIP preferably has the amino acid sequence set forth in SEQ ID NO: 2 or homologs, fragments or variants thereof.

**8.** The fusion protein of any one of claims 1 to 7, wherein A is an antigenic peptide comprising or consisting of an amino acid sequence selected from the group consisting of those set forth in SEQ ID NOs: 3, 8-10, 12-13.

**9.** The fusion protein of any one of claims 1 to 8, wherein "-" represents a linker comprising more than one amino acid and wherein the linker peptide forms a coiled coil structure.

**10.** The fusion protein of any one of claims 1 to 9, wherein the fusion protein has the amino acid sequence set forth in any one of SEQ ID NOs: 4-5, 11, 14-15.

**11.** A method for detecting a presence or amount of an analyte molecule, the method comprising:

contacting a fusion protein according to any one of claims 1 to 10 with the analyte molecule under conditions that allow binding of A to the analyte molecule; and
detecting the presence of the analyte molecule by determining a signal produced by the fusion protein:analyte complex.

**12.** A kit for detecting a presence of an analyte molecule or an amount of analyte, the kit comprising a fusion protein according to any one of claims 1 to 10 and optionally further comprising a substance for detecting the signal produced by the fusion protein.

**Patentansprüche**

**1.** Ein Fusionsprotein, das die Struktur

$$(A_n\text{-}B\text{-}A_x\text{-}C\text{-}A_m)_y$$

umfasst, wobei

jedes A unabhängig voneinander ein Peptid oder Protein-Domäne, die einen Analyten von Interesse binden, umfasst, wobei A einen Antikörper, ein Antikörperfragment, ein Peptid-Aptamer, ein Peptid-Antigen oder ein Peptid-Antigen-Fragment umfasst, wobei das Peptid-Antigen oder das Peptid-Antigen-Fragment vorzugsweise eine antigene Determinante oder ein Epitop umfassen;

B ein Enzym ist oder enzymatische Aktivität besitzt und eine Einheit umfasst, die ein nachweisbares Signal erzeugen kann;

C ein Inhibitor von B ist und eine Einheit umfasst, die, wenn A nicht an den Analyten von Interesse gebunden ist, an B bindet und das durch B erzeugte Signal moduliert;

n und m jeweils unabhängig voneinander 0 oder mindestens 1 sind;

x eine ganze Zahl von mindestens 1 ist;

y eine ganze Zahl von mindestens 1 ist;

"-" eine kovalente Bindung oder einen Linker repräsentiert, die aus einer oder mehreren Aminosäuren besteht oder diese umfasst;

wobei B und C jeweils unabhängig voneinander ein Peptid oder Protein-Domäne umfassen,

wobei die Länge der Verbindung zwischen den Peptiden oder den Proteindomänen derart ausgewählt ist, dass sie die Wechselwirkung von B und C ermöglicht, wenn A nicht an den Analyten von Interesse gebunden ist, und sie die Wechselwirkung von B und C verhindert, wenn A an den Analyten von Interesse gebunden ist.

2. Das Fusionsprotein nach Anspruch 1, wobei B ein Peptid oder eine Proteindomäne ist, die an eine Substanz gekoppelt ist, die das nachweisbare Signal erzeugen kann.

3. Das Fusionsprotein nach Anspruch 1 oder 2, wobei die Bindung von B und C beeinträchtigt ist, wenn alle A-Einheiten des Fusionsproteins an einen Analyten, gegebenenfalls den gleichen Analyten, gebunden sind.

4. Das Fusionsprotein nach einem der Ansprüche 1 bis 3, wobei das Fusionsprotein aus der Struktur

$$(B\text{-}A\text{-}C)_y \quad \text{besteht.}$$

besteht.

5. Das Fusionsprotein nach Anspruch 1, wobei B ein Chromophor oder Fluorophor umfasst und C eine Substanz umfasst, die Emissionsenergie des Chromophors oder Fluorophors absorbiert.

6. Das Fusionsprotein nach einem der Ansprüche 1 bis 5, wobei, wenn mehr als ein A in dem Fusionsprotein vorhanden ist, jedes A gleich oder verschieden voneinander ist, wobei, wenn die zwei oder mehr A verschieden sind, sie so angeordnet sind, dass sie den gleichen Analyten binden.

7. Das Fusionsprotein nach einem der Ansprüche 1 bis 6, wobei

(1) B beta-Lactamase TEM1 oder Homologe, Fragmente und Varianten davon ist, wobei die Homologe, Fragmente und Varianten wenigstens teilweise enzymatische Aktivität beibehalten, wobei die beta-Lactamase TEM1 vorzugsweise die Aminosäuresequenz von SEQ ID NO:1 oder Homologe, Fragmente oder Varianten davon umfasst; und

(2) C beta-Lactamase-Inhibitor-Protein (BLIP) oder Fragmente, Homologe und Varianten davon, die mindestens teilweise die Bindungsaktivität zur beta-Lactamase beibehalten, ist, wobei die BLIP vorzugsweise die Aminosäuresequenz von SEQ ID NO:2 oder Homologe, Fragmente oder Varianten davon aufweist.

8. Das Fusionsprotein nach einem der Ansprüche 1 bis 7, wobei A ein antigenes Peptid ist, das aus einer Aminosäuresequenz besteht oder eine solche umfasst, die aus der Gruppe bestehend aus denen wie in SEQ ID Nos. 3, 8-10, 12-13 ausgeführt, ausgewählt ist.

9. Das Fusionsprotein nach einem der Ansprüche 1 bis 8, wobei "-" eine Verbindung repräsentiert, die mehr als eine Aminosäure umfasst und wobei das Verbindungs-Peptid eine Coiled-Coil-Struktur bildet.

**10.** Das Fusionsprotein nach einem der Ansprüche 1 bis 9, wobei das Fusionsprotein die Aminosäuresequenz hat wie in einer von SEQ ID Nos. 4-5, 11, 14-15 ausgeführt.

**11.** Ein Verfahren zum Nachweis des Vorhandenseins oder einer Menge eines Analyt-Moleküls, wobei das Verfahren umfasst:

Kontaktieren eines Fusionsproteins nach einem der Ansprüche 1 bis 10 mit dem Analyt-Molekül unter Bedingungen, die A die Bindung des Analyt-Moleküls gestatten; und
Nachweisen der Anwesenheit des Analyt-Moleküls durch Bestimmen eines Signals, das durch den Fusionsprotein:Analyt-Komplex erzeugt wird.

**12.** Ein Kit zum Nachweis des Vorhandenseins eines Analyt-Moleküls oder einer Menge eines Analyten, das Kit umfasst ein Fusionsprotein nach einem der Ansprüche 1 bis 10 und gegebenenfalls ferner eine Substanz zum Nachweis des Signals, das durch das Fusionsprotein erzeugt wird.

**Revendications**

**1.** Protéine de fusion comprenant une structure de

$$(A_n\text{-}B\text{-}A_x\text{-}C\text{-}A_m)_y$$

dans laquelle

chaque A comprend indépendamment un peptide ou un domaine protéique qui se lie à un analyte d'intérêt, où A comprend un anticorps, un fragment d'anticorps, un aptamère peptidique, un antigène peptidique, ou un fragment d'antigène peptidique, ou l'antigène peptidique ou fragment d'antigène peptidique comprend de préférence un déterminant antigénique ou un épitope ;
B est une enzyme ou possède une activité enzymatique, et comprend une entité qui peut produire un signal détectable ;
C est un inhibiteur de B, et comprend une entité qui, lorsque A n'est pas lié à l'analyte d'intérêt, se lie à B et module la production d'un signal par B ;
n et m sont chacun indépendamment 0 ou au moins 1 ;
x est un nombre entier d'au moins 1 ;
y est un nombre entier d'au moins 1 ;
« - » représente une liaison covalente ou un segment de liaison comprenant ou consistant en un ou plusieurs acides aminés ;

où B et C comprennent chacun indépendamment un peptide ou un domaine protéique, où la longueur du segment de liaison entre les peptides ou les domaines protéiques est sélectionnée de sorte à permettre l'interaction de B et C lorsque A n'est pas lié à l'analyte d'intérêt, et à empêcher l'interaction de B et C lorsque A est lié à l'analyte d'intérêt.

**2.** Protéine de fusion selon la revendication 1, dans laquelle B est un peptide ou un domaine protéique couplé à une substance qui peut produire le signal détectable.

**3.** Protéine de fusion selon la revendication 1 ou 2, dans laquelle la liaison de B et C est altérée lorsque tous les fragments A de la protéine de fusion sont liés à un analyte, facultativement le même analyte.

**4.** Protéine de fusion selon l'une quelconque des revendications 1 à 3, où la protéine de fusion consiste en la structure

$$(B\text{-}A\text{-}C)_y.$$

**5.** Protéine de fusion selon la revendication 1, dans laquelle B comprend un chromophore ou un fluorophore et C comprend une substance qui absorbe l'énergie d'émission du chromophore ou fluorophore.

6. Protéine de fusion selon l'une quelconque des revendications 1 à 5, dans laquelle lorsque plus d'un seul A est présent dans la protéine de fusion, chaque A est le même ou différent, dans laquelle lorsque les deux A ou plus sont différents, ils sont arrangés pour se lier au même analyte.

7. Protéine de fusion selon l'une quelconque des revendications 1 à 6, dans laquelle

(1) B est la bêta-lactamase TEM1 ou des homologues, fragments et variants de celle-ci, où les homologues, fragments et variants conservent au moins partiellement l'activité enzymatique, où la bêta-lactamase TEM1 comprend de préférence la séquence d'acides aminés décrite dans SEQ ID NO: 1 ou des homologues, fragments ou variants de celle-ci ; et
(2) C est la protéine inhibitrice de la bêta-lactamase (BLIP) ou des fragments, homologues et variants de celle-ci qui conservent au moins partiellement l'activité de liaison pour la bêta-lactamase, où la BLIP possède de préférence la séquence d'acides aminés décrite dans SEQ ID NO: 2 ou des homologues, fragments ou variants de celle-ci.

8. Protéine de fusion selon l'une quelconque des revendications 1 à 7, dans laquelle A est un peptide antigénique comprenant ou consistant en une séquence d'acides aminés sélectionnée dans le groupe consistant en celles décrites dans SEQ ID NO: 3, 8-10, 12-13.

9. Protéine de fusion selon l'une quelconque des revendications 1 à 8, dans laquelle « - » représente un segment de liaison comprenant plus d'un seul acide aminé et dans laquelle le peptide de liaison forme une structure en super-rhélice.

10. Protéine de fusion selon l'une quelconque des revendications 1 à 9, où la protéine de fusion possède la séquence d'acides aminés décrite dans l'une quelconque de SEQ ID NO: 4-5, 11, 14-15.

11. Procédé pour détecter une présence ou quantité d'une molécule d'analyte, le procédé comprenant :

la mise en contact d'une protéine de fusion selon l'une quelconque des revendications 1 à 10 avec la molécule d'analyte dans des conditions qui permettent la liaison de A à la molécule d'analyte ; et
la détection de la présence de la molécule d'analyte en déterminant un signal produit par le complexe protéine de fusion:analyte.

12. Kit pour détecter une présence d'une molécule d'analyte ou une quantité d'analyte, le kit comprenant une protéine de fusion selon l'une quelconque des revendications 1 à 10 et comprenant en outre facultativement une substance pour détecter le signal produit par la protéine de fusion.

Figure 1(a)

Figure 1(b)

Figure 2

Figure 3

Figure 4

p53 pept/nutlin

= Enzyme (ß-lactamase)

= Gly-Ser linker

= enzyme inhibitor (BLIP)

= hdm2

= p53 peptide/nutlin

Figure 5

Figure 6

Figure 7

Figure 8

Figure 9

Figure 10

Figure 11

Figure 12

**REFERENCES CITED IN THE DESCRIPTION**

**Patent documents cited in the description**

- WO 0003727 A1

- SG 2011020872